# EUROPEAN PATENT SPECIFICATION

(11) **EP 3 099 813 B1**
(45) Date of publication and mention of the grant of the patent: **05.06.2019**
(21) Application number: 15702733.5
(22) Date of filing: 30.01.2015
(51) Int. Cl.: C12Q 1/689

(54) **GENETIC RESISTANCE TESTING**
PRÜFUNG DER GENETISCHEN WIDERSTANDSFÄHIGKEIT
TEST DE RÉSISTANCE GÉNÉTIQUE

(30) Priority: 30.01.2014 EP 14153260; 01.08.2014 EP 14179456
(43) Date of publication of application: 07.12.2016
(73) Proprietor: Ares Genetics GmbH, 1030 Vienna (AT)
(72) Inventor: KELLER, Andreas, 66346 Püttlingen (DE); KIRSTEN, Jan, 96049 Bamberg (DE); SCHMOLKE, Susanne, 91052 Erlangen (DE); STÄHLER, Cord, Friedrich, 69493 Hirschberg an der Bergstrasse (DE); RENSEN, Gabriel, Ukiah, CA 95482 (US); BACKES, Christina, 66125 Saarbrücken (DE)
(74) Representative: Schnappauf, Georg
(86) International application number: PCT/EP2015/051926
(87) International publication number: WO 2015/114094

(56) References cited:
- US-A1- 2005 069 897
- BARL T ET AL: "Genotyping DNA chip for the simultaneous assessment of antibiotic resistance and pathogenic potential of extraintestinal pathogenic Escherichia coli", INTERNATIONAL JOURNAL OF ANTIMICROBIAL AGENTS, ELSEVIER SCIENCE, AMSTERDAM, NL, vol. 32, no. 3, 1 September 2008 (2008-09-01), pages 272-277, XP024097956, ISSN: 0924-8579, DOI: 10.1016/J.IJANTIMICAG.2008.04.020 [retrieved on 2008-07-18]
- N. STOESSER ET AL: "Predicting antimicrobial susceptibilities for Escherichia coli and Klebsiella pneumoniae isolates using whole genomic sequence data", JOURNAL OF ANTIMICROBIAL CHEMOTHERAPY, vol. 4, 30 May 2013 (2013-05-30), XP55157002, ISSN: 0305-7453, DOI: 10.1093/jac/dkt180 cited in the application
- JIN D J ET AL: "Mapping and sequencing of mutations in the Escherichia colirpoB gene that lead to rifampicin resistance", JOURNAL OF MOLECULAR BIOLOGY, ACADEMIC PRESS, UNITED KINGDOM, vol. 202, no. 1, 5 July 1988 (1988-07-05), pages 45-58, XP024013372, ISSN: 0022-2836, DOI: 10.1016/0022-2836(88)90517-7 [retrieved on 1988-07-05]
- S. ADAMS-SAPPER ET AL: "Clonal Composition and Community Clustering of Drug-Susceptible and -Resistant Escherichia coli Isolates from Bloodstream Infections", ANTIMICROBIAL AGENTS AND CHEMOTHERAPY, vol. 57, no. 1, 12 November 2012 (2012-11-12), pages 490-497, XP055181744, ISSN: 0066-4804, DOI: 10.1128/AAC.01025-12

## Description

The invention relates to a method of determining an antibiotic resistance profile for a bacterial microorganism and to a method of determining the resistance of a bacterial microorganism to an antibiotic drug.

Antibiotic resistance is a form of drug resistance whereby a sub-population of a microorganism, e.g. a strain of a bacterial species, can survive and multiply despite exposure to an antibiotic drug. It is a serious and health concern for the individual patient as well as a major public health issue. Timely treatment of a bacterial infection requires the analysis of clinical isolates obtained from patients with regard to antibiotic resistance, in order to select an efficacious therapy.

Antibacterial drug resistance (ADR) represents a major health burden. According to the World Health Organization's antimicrobial resistance global report on surveillance, ADR leads to 25,000 deaths per year in Europe and 23,000 deaths per year in the US. In Europe, 2.5 million extra hospital days lead to societal cost of 1.5 billion euro. In the US, the direct cost of 2 million illnesses leads to 20 billion dollar direct cost. The overall cost is estimated to be substantially higher, reducing the gross domestic product (GDP) by up to 1.6%.

Currently, resistance / susceptibility testing is carried out by obtaining a culture of the suspicious bacteria, subjecting it to different antibiotic drug protocols and determining in which cases bacteria do not grow in the presence of a certain substance. In this case the bacteria are not resistant (i.e. susceptible to the antibiotic drug) and the therapy can be administered to the respective patients. The document US7335485 describes a method of determining the antibiotic susceptibility of a microorganism, wherein the organism is cultured in the presence of an antibiotic drug to be tested. More recently, sensitive technologies as Mass Spectrometry are applied to determine resistance, but this still requires culturing of the microorganism to be tested in the presence of an antibiotic drug to be tested. Further, in all these techniques, each microorganism to be tested has to be tested against individual antibiotic drugs or drug combinations, requiring extensive, time-consuming and cumbersome tests.

It is known that drug resistance can be associated with genetic polymorphisms. This holds for viruses, where resistance testing is established clinical practice (e.g. HIV genotyping). More recently, it has been shown that resistance has also genetic causes in bacteria and even higher organisms, such as humans where tumors resistance against certain cytostatic agents can be linked to genomic mutations.

Wozniak et al. (BMC Genomics 2012, 13(Suppl 7) :S23) disclose genetic determinants of drug resistance in Staphylococcus aureus based on genotype and phenotype data. Stoesser et al. disclose prediction of antimicrobial susceptibilities for Escherichia coli and Klebsiella pneumoniae isolates using whole genomic sequence data (J Antimicrob Chemother 2013; 68: 2234-2244).

Barl et al. (International Journal of Antimicrobial Agents 32 (2008) 272-277) report a study presenting the integration and application of two DNA chip modules for the simultaneous detection of single nucleotide polymorphisms in *gyrA* (quinolone resistance) and *fimH* (increased adhesion to urinary tract epithelium).

US 2005/0069897 A1 relates to a method for detecting quinolone-resistant *E*. *coli* strains in a biological sample material. As discussed therein, a number of clinical isolates of *E*. *coli* known to be quinolone resistant have been investigated.

Jin and Gross (J. Mol. Biol. (1988) 202, 45-58) describe ri-fampicin resistance and related *rpoB* gene mutation in *Escherichia coli.*

Adams-Sapper et al. (Antimicrob Agents Chemother. 2013 Jan;57(1):490-7) compare the clonal composition of drug-resistant and -susceptible *E*. *coli* clinical isolates.

Escherichia coli (E. coli) is a Gram-negative, facultative anaerobic, rod-shaped bacterium generally found e.g. in the lower gastro-intestinal tract of mammals. While many species of the Escherichia genus are harmless, some strains of some species are pathogenic in humans causing urinary tract infections, gastrointestinal disease, as well as a wide range of other pathologic conditions. E.coli is responsible for the majority of these pathologic conditions.

There remains a need for quick and efficient antibiotic resistance testing.

This need is addressed by the present invention.

### Summary of the Invention

The inventors performed extensive studies on the genome of E. coli bacteria resistant to antibiotic drugs and found remarkable differences to wild type E. coli. Based on this information, it is now possible to provide a detailed analysis on the resistance pattern of E. coli strains based on individual genes or mutations on a nucleotide level. This analysis involves the identification of a resistance against individual antibiotic drugs as well as clusters of them. This allows not only for the determination of a resistance to a single antibiotic drug, but also to groups of antibiotics such as lactam or quinolone antibiotics, or even to all relevant antibiotic drugs.

Therefore, the present invention will considerably facilitate the selection of an appropriate antibiotic drug for the treatment of an E. coli infection in a patient and thus will largely improve the quality of diagnosis and treatment.

The present invention is defined by the appended claims.

In an embodiment of the invention, the presence of a mutation in a gene selected from the group consisting of potB, ycgK, ycgB, valS and yjjJ is determined.

In an embodiment of the invention, the method comprises determining the presence of a mutation in more than one gene selected from said group.

In an embodiment of the invention, the presence of a mutation in a gene selected from said group is indicative of a resistance to antibiotic drugs belonging to different groups of antibiotic drugs.

In an embodiment of the invention, the mutation in the gene potB is L267V. Preferably, the mutation is indicative of a resistance to ciprofloxacin (CP), levofloxacin (LVX), and tetracycline (TE).

In an embodiment of the invention, the mutation in the gene ycgK is H63Q. Preferably, the mutation is indicative of a resistance to ciprofloxacin (CP), levofloxacin (LVX), cefazolin (CFZ), tetracycline (TE), and cefuroxime (CRM).

In an embodiment of the invention, the mutation in the gene ycgB is I497L. Preferably, the mutation is indicative of a resistance to ciprofloxacin (CP), levofloxacin (LVX), cefazolin (CFZ), and tetracycline (TE).

In an embodiment of the invention, the mutation in the gene valS is selected from the group consisting of D469E, N444K, Y439*, Y413*, K277N, D244E, Y242* and D176E, wherein the asterisk designates a non-conservative mutation leading to a new stop codon. Preferably in this embodiment, a mutation selected from the group consisting of D469E, N444K, Y439*, Y413*, K277N, and D176E is indicative of a resistance to ciprofloxacin (CP), levofloxacin (LVX), and cefazolin (CFZ). Also preferably in this embodiment, the mutation D244E is indicative of a resistance to ciprofloxacin (CP), levofloxacin (LVX), and gentamicin (GM) and the mutation Y242* is indicative of a resistance to ciprofloxacin (CP), levofloxacin (LVX), cefazolin (CFZ), and tetracycline (TE).

In an embodiment of the invention, the mutation in the gene yjjJ is H184R. Preferably, the mutation is indicative of a resistance to ciprofloxacin (CP), levofloxacin (LVX), and cefazolin (CFZ).

In any embodiment of the invention, the presence of a mutation in a gene selected from said group is tested in relation to the reference strain E. coli K12 substrain DH10B.

The present disclosure additionally describes a method of determining an antibiotic resistance profile for a bacterial microorganism belonging to the species E. coli comprising the steps of
a) providing a sample containing or suspected of containing the bacterial microorganism;
b) determining the presence of a mutation in at least one gene of the bacterial microorganism selected from the group of genes listed in Table 4;
wherein the presence of a mutation is indicative of a resistance to an antibiotic drug.

**Table 4 is depicted in the following:**

| | | | | | | | |
|---|---|---|---|---|---|---|---|
| abgB | mhpA | ybfD | yfcO | yncG | torZ | fadA | thiE |
| allA | mnmC | ybfQ | yfdF | yneK | uidB | fdx | thiM |
| argI | mukB | ycbF | yfdR | yphG | ycjX | fhuB | trpC |
| caiC | norW | ycbS | yfdX | zraS | ydiU | fhuC | udp |
| csiE | ompA | yccE | yfhM | agaD | yejA | fhuD | uxaA |
| cynX | ompC | yceH | ygbN | astE | yfbL | fmt | ybiB |
| dadX | parC | ycgB | ygcQ | chbG | yfiK | gudP | ybiU |
| elaD | pgaA | ycgK | ygeK | eutE | ygcR | helD | ydfI |
| fcl | potB | yciQ | ygeO | eutQ | ygcU | hrpB | ydgA |
| fhuA | puuC | ydbD | ygiD | flgL | ygfZ | ilvA | yecA |
| flhA | puuE | yddV | yhaC | gcvP | ygiF | kdpD | yehT |
| flu | rem | ydeK | yhaI | gspO | ygjM | ldcA | yfcN |
| frwC | rhsC | ydjO | yhdP | gudD | yjjU | lplA | yheN |
| gyrA | rhsD | yeaU | yhgE | hemF | yjjW | menB | yhgF |
| hofB | Rz | yeaX | yhiJ | kdpE | yohG | metH | yhhQ |
| htrL | stfR | yeeJ | yjbI | ldrA | ypdB | pbpC | yhjE |
| hybA | tilS | yefM | yjcF | livG | yqjA | purH | yjjG |
| hyfB | valS | yegE | yjfF | murB | yrfB | purK | ynfA |
| hyfG | xseA | yegI | yjfZ | murP | ytfG | purL | |
| hyfI | yacH | yehB | yjgL | nepI | aspS | queF | |
| hypF | yafE | yehI | yjgN | pphB | birA | rhaA | |
| ilvY | yafT | yehM | yjhS | ptrB | cysD | rhaB | |
| lsrC | yagR | yeiI | yjjJ | rhaD | dapB | rplO | |
| lsrF | yaiO | yfaL | ymdC | speC | dxs | srlD | |
| menE | ybbB | yfaW | ynbB | tiaE | eutA | thiC | |

The presence or absence of a mutation in these genes is tested in relation to the reference strain E. coli K12 substrain DH10B (see also more detailed information in the following and in Example 1). In a preferred embodiment of the disclosure, step b) comprises determining the presence of a mutation in at least two or more genes selected from the group of Table 4, and wherein the presence of a mutation in at least two genes is indicative of a resistance to an antibiotic drug.

Instead of testing only single genes or mutants, a combination of several variant positions can improve the prediction accuracy and further reduce false positive findings that are influenced by other factors. Therefore, it is in particular preferred to determine the presence of a mutation in 2, 3, 4, 5, 6, 7, 8 or 9 (or more) genes selected from Table 4.

In a further preferred embodiment of the disclosure, the present method comprises in step b) determining the presence of a mutation in at least one gene selected from the group of genes listed in Table 5, and wherein the presence of a mutation in said at least one gene is indicative of a resistance to an antibiotic drug.

The genes according to Table 5 have never been described before in the context of antibiotic resistance of E. coli bacteria. They may be used for the determination of an antibiotic drug resistance of E. coli alone or in combination with other genes disclosed herein.

| | | | | | | | |
|---|---|---|---|---|---|---|---|
| abgB | yegI | ymdC | ycjX | | ldcA | | yhjE |
| frwC | yehM | ynbB | ydiU | | lplA | | yjjG |
| hofB | yeiI | yncG | yfbL | | menB | | |
| htrL | yfaW | yneK | yfiK | | metH | | |
| hybA | yfcO | yphG | ygcR | | pbpC | | |
| hyfB | yfdF | zraS | ygcU | | purH | | |
| hyfI | yfdR | agaD | ygfZ | | purL | | |
| lsrF | yfdX | chbG | ygiF | | queF | | |
| potB | ygbN | eutE | ygjM | | rhaB | | |
| puuC | ygcQ | eutQ | yjjU | | rplO | | |
| yafT | ygeK | flgL | yjjW | | srlD | | |
| yagR | ygeO | gcvP | yohG | | thiC | | |
| yaiO | ygiD | gspO | yqjA | | thiE | | |
| ybbB | yhaC | gudD | yrfB | | thiM | | |
| ybfD | yhgE | hemF | ytfG | | uxaA | | |
| ybfQ | yhiJ | ldrA | aspS | | ybiB | | |
| ycbF | yjbI | livG | cysD | | ybiU | | |
| ycbS | yjcF | murP | eutA | | ydfI | | |
| ycgB | yjfF | nepI | fadA | | ydgA | | |
| ycgK | yjfZ | pphB | fdx | | yecA | | |
| yciQ | yjgL | ptrB | fhuC | | yehT | | |
| yddV | yjgN | | rhaD | gudP | | yfcN | |
| ydjO | yjhS | | tiaE | helD | | yheN | |
| yeaX | yjjJ | | torZ | hrpB | | yhgF | |
| yeeJ | ynfA | | uidB | kdpD | | yhhQ | |

For E.coli 86 ultra highly significant pairs of genetic positions and drug resistance (table 2) were identified. The 86 combinations correspond to 35 genetic positions, since the sites are usually significant for more than one single drug. Most importantly, the respective sites are located in 9 genes: hofB, allA, mukB, ymdC, potB, ycgK, ycgB, valS, yjjJ. These genes thus appear to be critical for antibiotic resistance/susceptibility. The identified mutations all lead to amino acid alterations, either to an exchange of amino acid at the respective position or the creation of a new stop-codon. For more detailed information, it is referred to Example 1, below.

More generally, the present disclosure in a further aspect relates to a method of determining the resistance or susceptibility of a bacterial microorganism belonging to the species E. coli to an antibiotic drug comprising:
- providing a sample containing or suspected of containing the bacterial microorganism belonging to the species E. coli;
- determining from said sample a nucleic acid sequence information of at least one gene selected from the group of hofB, allA, mukB, ymdC, potB, ycgK, ycgB, valS, and yjjJ; and
- based on the determination of said genetic information determining the resistance or susceptibility to the antibiotic drug.

In a further embodiment of the disclosure, the presence of a mutation in at least one gene selected from the group of hofB, allA, mukB, ymdC, potB, ycgK, ycgB, valS, and yjjJ is determined. Thus, the presence of a mutation in at least one or 2, 3, 4, 5, 6, 7, 8 or 9 of these genes can be analyzed.

In a further embodiment of the disclosure, the presence of a mutation in at least one gene selected from the group of the following table 6 is determined. More preferably, the exact amino acid exchange indicated in Table 6 is determined.

| Gene Name | Amino Acid Exchange | Gene Name | Amino Acid Exchange |
|---|---|---|---|
| aspS | D382E | purK | N137D |
| birA | Q113H | purL | D615E |
| cysD | D232N | queF | K126E |
| dapB | N87K | rhaA | S406N |
| dxs | A541T | rhaB | T407A |
| eutA | A210V | rplO | K39N |
| fadA | V387I | srlD | M54T |
| fdx | S66T | thiC | H193R |
| fhuB | G448V | thiE | A121E |
| fhuC | A122V | thiE | R43Q |
| fhuD | D76E | thiM | A122T |
| fmt | V30I | trpC | L378F |
| gudP | A448V | udp | I147M |
| gyrA | D87N;D87Y | uxaA | E236A |
| helD | E671D | ybiB | G35S |
| hrpB | A413T | ybiU | M419I |
| hrpB | V240A | ydfI | A146V |
| ilvA | D401E | ydgA | F416L |
| kdpD | E376D | yecA | I195V |
| ldcA | R167Q | yehT | A106V |
| lplA | A279T | yfcN | I39V |
| menB | T31A | yheN | Q49H |
| metH | E1124;E1124D | yhgF | E737D |
| mukB | S1015N | yhhQ | R138H |
| parC | S80I | yhjE | I323V |
| parC | S80R | yjjG | A57V |
| pbpC | H37Q | ynfA | T84S |
| purH | T366I | | |

Surprisingly, the inventors found that an overlap of mutations in functionally similar proteins of E. coli and K. pneumoniae exists. Interestingly, when considering the proteins that were associated significantly with at least one drug, the inventors found an overlap of 1,746 proteins (same official name and more than 80 percent positives in BLAST in pairwise comparison) that are affected in E. coli as well as in K. pneumoniae. Extending the analysis to the exact AA exchanges in these proteins, the inventors still detected an overlap of 55 mutated positions that are equal in both organisms. Therefore, the above genes might form a valuable basis for the determination of the antibiotic resistance pattern in both, E. coli and K. pneumonia microorganisms.

According to an optional aspect of the disclosure, the nucleic acid sequence information can be the determination of the presence of a single nucleotide at a single position in at least one gene.

Thus the disclosure comprises a method wherein the presence of a single nucleotide polymorphism or mutation at a single nucleotide position is detected.

For example, this can be done in at least one gene selected from the group of hofB, allA, mukB, ymdC, potB, ycgK, ycgB, valS, and yjjJ. Therefore, according to an optional aspect of the disclosure, the mutation is a mutation which is selected from the group of mutations listed in table 2 (see below in Example 1). The present disclosure thus also includes a method of determining an antibiotic resistance profile for a bacterial microorganism belonging to the species E. coli comprising the steps of
a) providing a sample containing or suspected of containing the bacterial microorganism;
b) determining the presence of a mutation in at least one position as identified in Table 2;
wherein the presence of a mutation is indicative of a resistance to an antibiotic drug.

The determination can be made based on 1, 2, 3, 4, 5, 6, 7, and up to the 35 genetic positions identified in Table 2.

Generally, the method according to the present disclosure involves determining the resistance of E. coli to one or more antibiotic drugs. These drugs include, but are not restricted to antibiotic drugs selected from the group consisting of ampicillin sulbactam (A.S.), ampicillin (AM), amoxicillin clavulanate (AUG), aztreonam (AZT), ceftriaxone (CAX), ceftazidime (CAZ), cefotaxime (CFT), cefepime (CPM), ciprofloxacin (CP), ertapenem (ETP), levofloxacin (LVX), cefuroxime (CRM), piperazillin tazobactam (P/T), trimethoprim sulfameth-oxazole (T/S), tobramycin (TO), gentamicin (GM), cefazolin (CFZ), cephalotin (CF), imipenem (IMP), meropenem (MER) and tetracycline (TE). See also Table 1.

The inventors have surprisingly found that mutations in certain genes are indicative not only for a resistance to one single antibiotic drug, but to groups containing several drugs.

For example, it turned out that in case of the group of lactam antibiotics, the presence of a mutation in the following genes: chbG, eutQ, flgL, gudD, gyrA, ldrA, menE, murB, murP, nepI, parC, pphB, ptrB, rhaD, ydiU, yegE, yegI, yfbL, yfiK, ygcR, ygiF, ygjM, yohG, and/or yrfB should be determined and is indicative for the presence of a resistance against antibiotics of this group.

The group of lactam antibiotics preferably comprises A.S., AM, AUG, AZT, CFZ, CPE, CFT, CAZ, CAX, CRM, CF, CP, IMP, MER, ETP and/or P/T. The p-value threshold for these identified genes is ≤ 10⁻⁴⁵.

It is within the scope of the present disclosure that the above determination is done based on a single gene or 2, 3, 4 etc. genes of this group, however, it is preferred to determine a mutation in all of these genes in relation to the reference strain K12 substrain DH10B (see also below for further information).

In a further embodiment of the present disclosure, the antibiotic drug is selected from quinolone or aminoglycoside antibiotics and the presence of a mutation in the following genes is determined: agaD, chbG, eutE, eutQ, gcvP, gspO, gyrA, livG, menE, nepI, parC, speC, tiaE, torZ, uidB, yegE, yegI, yejA, ygcU, ygfz, ygiF, ygjM, yjjU, yjjW, ymdC, ypdB, yqjA, and/or ytfG.

The quinolone and aminoglycoside antibiotics preferably are selected from CP, LVX, GM and TO.

Surprisingly, the relevant genes completely overlapped regarding a resistance to quinolone and aminoglycoside antibiotics; the p-value threshold for these genes is ≤ 10⁻⁵³. Also here, it is within the scope of the present disclosure that the determination is done based on a single gene or in 2, 3, 4 or more genes of this group only, however, it is preferred to determine a mutation in all of these genes in relation to the reference strain K12 substrain DH10B.

In a further embodiment of the present disclosure, the antibiotic drug is selected from tetracycline and the presence of a mutation in at least one or more of the following genes is determined: astE, chbG, eutQ, flgL, gudD, gyrA, hemF, hypF, kdpE, ldrA, menE, murB, murP, nepI, ompC, parC, pphB, ptrB, and/or rhaD. The p-value threshold is ≤ 10⁻⁴⁷.

In a still further embodiment of the present disclosure, the antibiotic drug is selected from trimethoprim sulfmethoxazol and the presence of a mutation in at least one or more of the following genes is determined: astE, chbG, eutQ, flgL, gudD, gyrA, ldrA, menE, murB, nepI, parC, ycjx, ydiU, yegE, yfiK, ygcR, ygiF, and/or yrfB. The p-value threshold is ≤ 10⁻⁴⁸.

In a preferred embodiment of the present disclosure, the method of the present disclosure comprises determining a mutation, wherein the mutation is selected from the group of mutations listed in Table 7. Table 7 is depicted in the following:

| Genome Pos | Therapy | Ref | Alt | AA | Alt AA | Gene | Exchange |
|---|---|---|---|---|---|---|---|
| 37032 | P/T | C | T | V | I | caiC | V270I |
| 206427 | AM | C | A, G | T | R, K | yafE | T133R;T133K |
| 319290 | P/T | A | T | D | E | yaiO | D36E |
| 1181357 | AM | C | T | T | M | yceH | T178M |
| 1368519 | P/T | G | T | AA | S | - | A137S |
| 1516808 | P/T | G | A, T | AA | T, S | stfR | A114T;A114S |
| 1517573 | P/T | G | C | E | Q | stfR | E369Q |
| 1567286 | P/T | G | A | AA | T | ynbB | A148T |
| 1615473 | AZT, CAX | A | C, T | E | V, A | yncG | E203V;E203A |
| 1684413 | AM | C | T | M | I | ydeK | M441I |
| 1974644 | A/S | T | A, C | C | R, S | yeaX | C69R;C69S |
| 2052365 | ETP | A | T, C | I | Stop codon, M | flhA | 1427;I427M |
| 2178525 | P/T | C | T | G | D | yefM | G74D |
| 2216164 | P/T | C | T, G | R | K, T | fcl | R20K;R20T |
| 2233638 | ETP, P/T | G | A, T | L | F, Stop codon | yegE | L447F;L447 |
| 2428172 | CP, LVX | C | A, T | D | N, Y | gyrA | D87N;D87Y |
| 2428183 | A/S, AM, | G | A | S | L | gyrA | S83L |
| | AZT, | | | | | | |
| | CAX, | | | | | | |
| | CAZ, | | | | | | |
| | CFT, | | | | | | |
| | CPE, | | | | | | |
| | CRM, GM, | | | | | | |
| | T/S, TO | | | | | | |
| 2463877 | AUG | A | G | V | A | menE | V46A |
| 2565236 | ETP | G | T, A | A | S, T | yfdR | A156S;A156T |
| 2725302 | P/T | G | A | M | I | xseA | M428I |
| 2755319 | P/T | T | C | M | T | csiE | M33T |
| 2924554 | P/T | A | T | T | S | norW | T27S |
| 3240296 | P/T | G | A, C | F | Stop codon, L | hybA | F204;F204L |
| 4054212 | A/S | C | A, T | E | Stop codon, D | ilvY | E184;E184D |
| 4525576 | AM | T | C | I | V | yjfZ | I78V |
| 4553471 | ETP | C | A, T | L | I, F | yjfF | L20I;L20F |
| 4575887 | P/T | T | C, G | L | R, P | yjgL | L207R;L207P |
| 4636902 | AM | G | A | A | V | - | A175V |

The inventors found that, apart from the above genes indicative of a resistance against antibiotics, also single nucleotide polymorphisms (= SNP's) may have a high significance for the presence of a resistance against defined antibiotic drugs. The analysis of these polymorphisms on a nucleotide level may further improve and accelerate the determination of a drug resistance to antibiotics in E. coli.

For example, a resistance of E. coli against the antibiotic drug AM can be determined by the presence of a single nucleotide polymorphism in at least one, for example 1, 2, 3, 4, 5 or 6 of the following nucleotide positions: 2428183, 4525576, 1684413, 4636902, 1181357, 206427.

In an embodiment of the present disclosure, the antibiotic drug is A/S and an SNP in at least one, for example 1, 2 or 3 of the following nucleotide positions is detected: 2428183, 4054212, 1974644.

In a further embodiment of the present disclosure, the antibiotic drug is AUG and a mutation in the following nucleotide position is detected: 2463877.

For a resistance to the antibiotic drug AZT, a mutation in at least one of the following nucleotide positions is detected: 2428183, 1615473.

In a still further preferred embodiment of the present disclosure, the antibiotic drug is CAX and a mutation in at least one of the following nucleotide positions is detected: 2428183, 1615473.

A resistance to the antibiotic drugs CFT, CP, CPE, CRM, GM, LVX, TO, T/S or CAZ can be detected by a mutation in the nucleotide position 2428183.

When the antibiotic drug is ETP, a mutation in at least one, for example 1, 2, 3, or 4 of the following nucleotide positions is detected: 2052365, 2233638, 4553471, 2565236.

In a further embodiment of the present disclosure, the antibiotic drug is P/T and a mutation in at least one, for example 1, 2, 3, 4, 5, 6, 7, 8, 9, 10, 11, 12, 13, or 14 of the following nucleotide positions is detected: 2233638, 2216164, 2725302, 1567286, 2755319, 319290, 3240296, 1517573, 2178525, 2924554, 1516808, 37032, 1368519, 4575887.

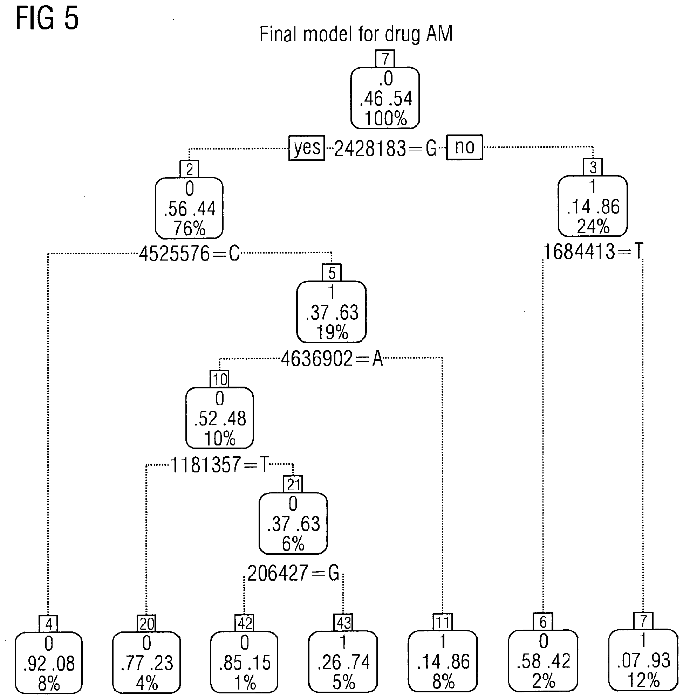

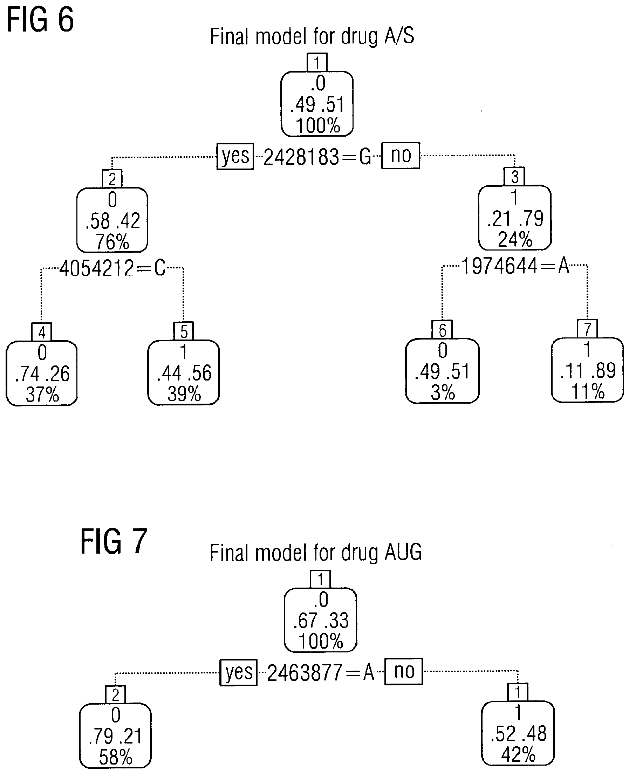

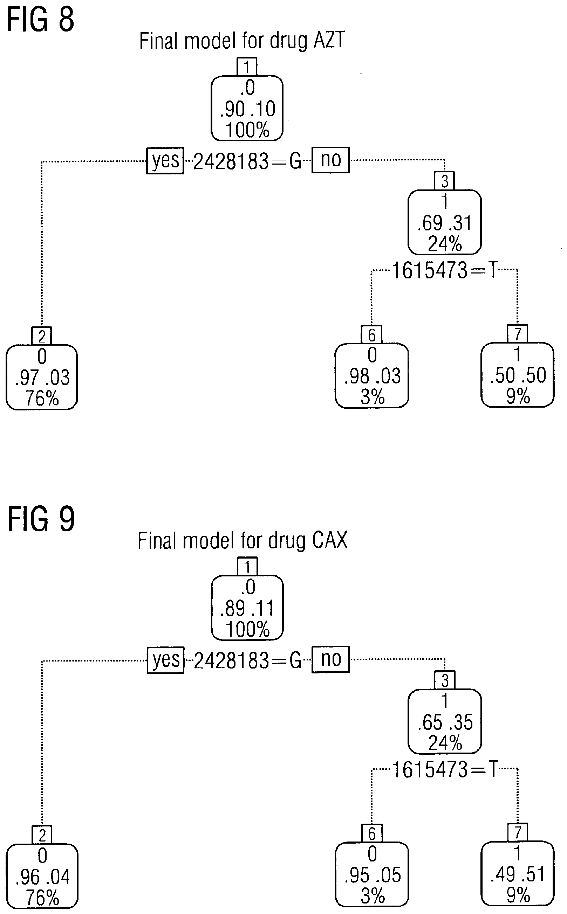

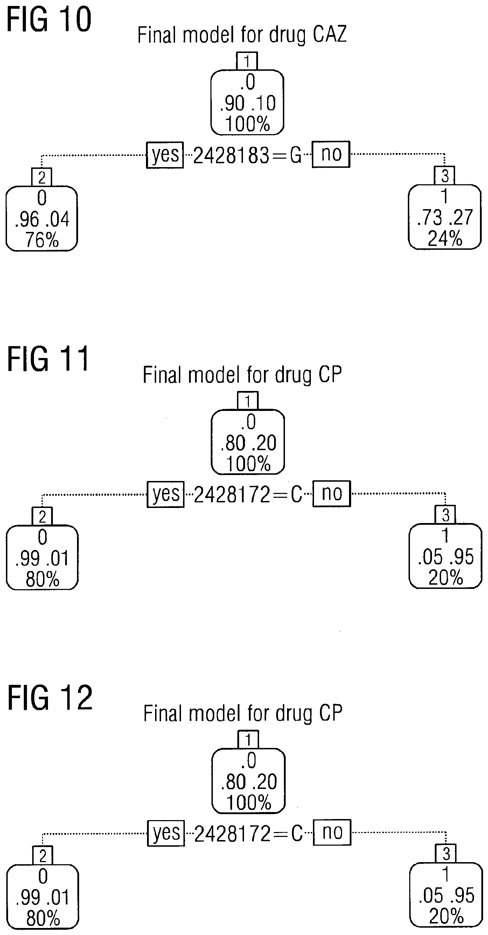

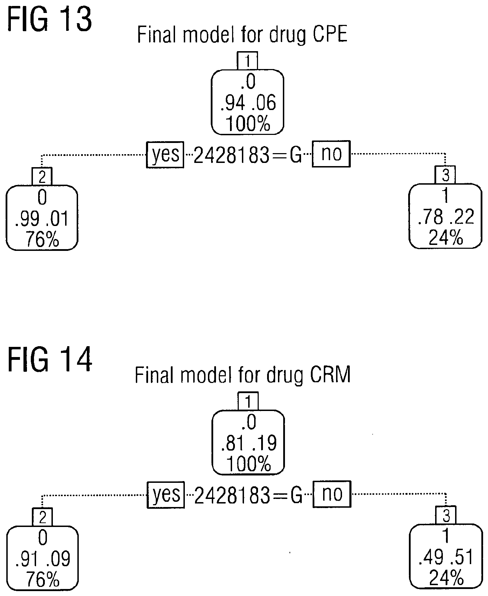

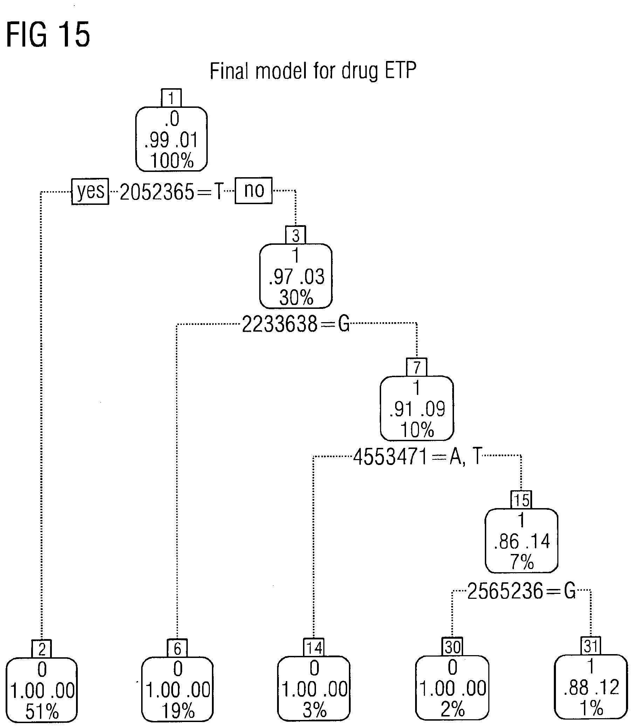

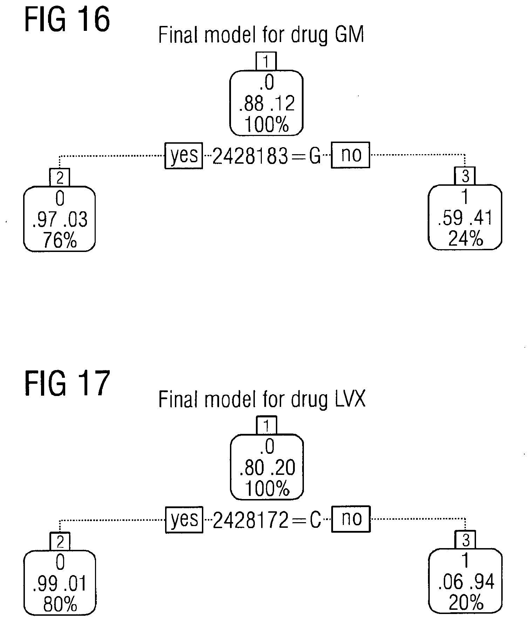

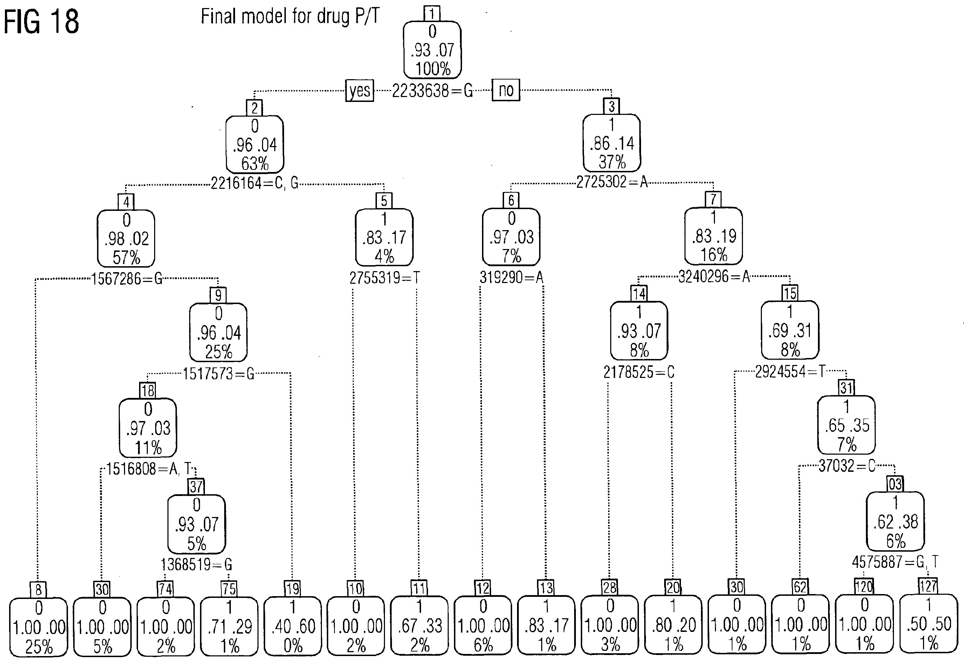

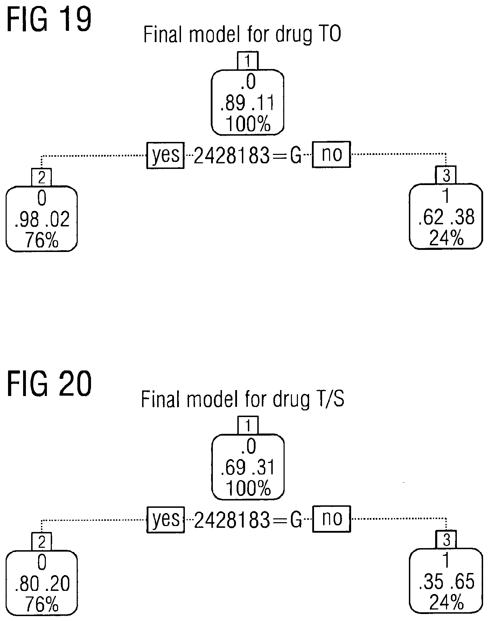

Preferably, the resistance to the respective antibiotic drug is tested according to the decision diagrams of Figures 5-20.

A decision diagram or "decision tree" is a tree-like graph for prediction tasks, e.g. classification. Given a data set consisting of a number of samples with feature values (any measurements, here SNPs) and class labels (here resistant/not resistant against a certain drug), a decision tree models the decision process of inferring the sample class label from its feature values.

To build the model a given data set is used (as described above): Among all features (SNPs) and their possible values (DNA bases A, C, T, and G) the feature value is selected which achieves the optimal sample separation with respect to the given sample labels. Ideally, that would be a SNP whose value for not resistant samples would be different as for the resistant samples. The selected feature value becomes the root of the tree (the first tree node, often drawn at the top) and the samples are split according to that feature, i.e. samples having that feature value and samples with another value. The resulting subsets of samples form new nodes and the feature selection and splitting process is repeated for each of them separately. This procedure stops if a specific criterion is fulfilled (e.g. no further improvement or maximal tree size is achieved).

The used graphical representation is defined as follows:
The tree root is always drawn at the top. Each node contains following information:
- Its feature and its value(s) drawn below the node, e.g. SNP 2428183 = G.
- Class label: 0 = not resistant, 1 = resistant.
- Class distribution: The proportion of samples contained in that node belonging to class 0 or 1.
- Proportion of samples contained in that node (w.r.t. to sample number used to build the tree).
- Color: green = 0, blue = 1, the stronger the color the higher the certainty for the chosen class label.

Generally, the model is built on the so-called training set and its prediction power is tested on the so-called test set (to assess the model performance on unseen data). Both data sets should be independent and have no intersection. However, if the available data set is not large enough to form a sufficient large training and test data sets, we apply a procedure called k-fold cross validation (CV): We divide our data set into k subsets of equal size, then each of the k subsets is used once as test data and the rest as training data. The final tree is built on the whole data set, so the CV is only used to estimate the performance of the final model.

The classification of a new sample works as follows:
- One starts at the tree root: the value of the root attribute in the sample is checked. If the value is equal to the root value then one goes left to the next node. Otherwise, one goes right.
- The value of the current node attribute in the sample is checked and it is decided again whether to go left or right. And so on.
- The process stops if one is in a leaf node (terminal node, node without outgoing edges). The sample gets the same label as that leaf node.

According to an optional aspect of the disclosure, a detected mutation is a mutation leading to an altered amino acid sequence in a polypeptide derived from a respective gene in which the detected mutation is located. According to this aspect, the detected mutation thus leads to a truncated or version of the polypeptide (wherein a new stop codon is created by the mutation) or a mutated version of the polypeptide having an amino acid exchange at the respective position.

According to an optional aspect of the disclosure, determining the nucleic acid sequence information or the presence of a mutation comprises determining a partial sequence or an entire sequence of the at least one gene.

According to an optional aspect of the disclosure, determining the nucleic acid sequence information or the presence of a mutation comprises determining a partial or entire sequence of the genome of said bacterial microorganism, wherein said partial or entire sequence of the genome comprises at least a partial sequence of said at least one gene.

According to an optional aspect of the disclosure the sample is a patient sample (clinical isolate).

According to an optional aspect of the disclosure determining the nucleic acid sequence information or the presence of a mutation comprises a using a next generation sequencing or high throughput sequencing method. According to a preferred further aspect of this aspect of the disclosure, a partial or entire genome sequence of the bacterial organism is determined by a using a next generation sequencing or high throughput sequencing method.

According to an optional aspect of the disclosure, the method of the disclosure further comprises determining the resistance to 2, 3, 4, 5, or 6 antibiotic drugs.

In a further aspect, the present disclosure is directed to a diagnostic method of determining an antibiotic resistant E. coli infection in a patient, comprising the steps of:
a) obtaining or providing a sample containing or suspected of containing E. coli from the patient;
b) determining the presence of at least one mutation in at least one gene as described above, wherein the presence of said at least one mutation is indicative of an antibiotic resistant E. coli infection in said patient.

In a still further aspect, the present disclosure is directed to a method of treating a patient suffering from an antibiotic resistant E. coli infection in a patient:
a) obtaining or providing a sample containing or suspected of containing E. coli from the patient;
b) determining the presence of at least one mutation in at least one gene as described above, wherein the presence of said at least one mutation is indicative of a resistance to one or more antibiotic drugs;
c) identifying said at least one or more antibiotic drugs;
d) selecting one or more antibiotic drugs different from the ones identified in step c) and being suitable for the treatment of an E. coli infection; and
e) treating the patient with said one or more antibiotic drugs.

According to a preferred embodiment of the present disclosure, the patient is a vertebrate, more preferably a mammal and most preferred a human patient.

Regarding the dosage of the antibiotic drug, it is referred to the established principles of pharmacology in human and veterinary medicine. For example, Forth, Henschler, Rummel "Allgemeine und spezielle Pharmakologie und Toxikologie", 9th edition, 2005 might be used as a guideline. Regarding the formulation of a ready-to-use medicament, reference is made to "Remington, The Science and Practice of Pharmacy", 22nd edition, 2013.

### Definitions

Unless defined otherwise, technical and scientific terms used herein have the same meaning as commonly understood by one of ordinary skill in the art to which this disclosure belongs.

The term "nucleic acid molecule" refers to a polynucleotide molecule having a defined sequence. It comprises DNA molecules, RNA molecules, nucleotide analog molecules and combinations and derivatives thereof, such as DNA molecules or RNA molecules with incorporated nucleotide analogs or cDNA.

The term "nucleic acid sequence information" relates to an information which can be derived from the sequence of a nucleic acid molecule, such as the sequence itself or a variation in the sequence as compared to a reference sequence.

The term "mutation" relates to a variation in the sequence as compared to a reference sequence. Such a reference sequence can be a sequence determined in a predominant wild type organism or a reference organism, e.g. a defined and known bacterial strain or substrain. A mutation is for example a deletion of one or multiple nucleotides, an insertion of one or multiple nucleotides, or substitution of one or multiple nucleotides, duplication of one or a sequence of multiple nucleotides, translocation one or a sequence of multiple nucleotides, and, in particular, a single nucleotide polymorphism (SNP).

In the context of the present disclosure a "sample" is a sample which comprises nucleic acid molecule from a bacterial microorganism. Examples for samples are: cells, tissue, body fluids, biopsy specimens, blood, urine, saliva, sputum, plasma, serum, cell culture supernatant, swab sample and others.

New and highly efficient methods of sequencing nucleic acids referred to as next generation sequencing have opened the possibility of large scale genomic analysis. The term "next generation sequencing" or "high throughput sequencing" refers to high-throughput sequencing technologies that parallelize the sequencing process, producing thousands or millions of sequences at once. Examples include Massively Parallel Signature Sequencing (MPSS) Polony sequencing, 454 pyrosequencing, Illumina (Solexa) sequencing, SOLiD sequencing, Ion semiconductor sequencing, DNA nanoball sequencing, Helioscope(TM) single molecule sequencing, Single Molecule SMRT(TM) sequencing, Single Molecule real time (RNAP) sequencing, Nanopore DNA sequencing.

Before the it is described in exemplary detail, it is to be understood that this disclosure is not limited to the particular component parts of the process steps of the methods described herein as such methods may vary. It is also to be understood that the terminology used herein is for purposes of describing particular embodiments of the present disclosure only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

### Figure legends

Figures 1-4 are related to Example 2, below.
Figure 1: An exemplary contingency table for the computation of the Fisher's exact test and the measures accuracy, sensitivity, specificity, positive predictive value (PPV), and negative predictive value (NPV). Numbers are given for amino acid exchange S83L (GyrA) and Ciprofloxacin.
Figure 2: Overview of mean MIC values for Ciprofloxacin for samples having no mutation in GyrA (S83, D87) and ParC (S80), either one mutation in GyrA and not ParC, both mutations in GyrA and not ParC, or all three mutations.

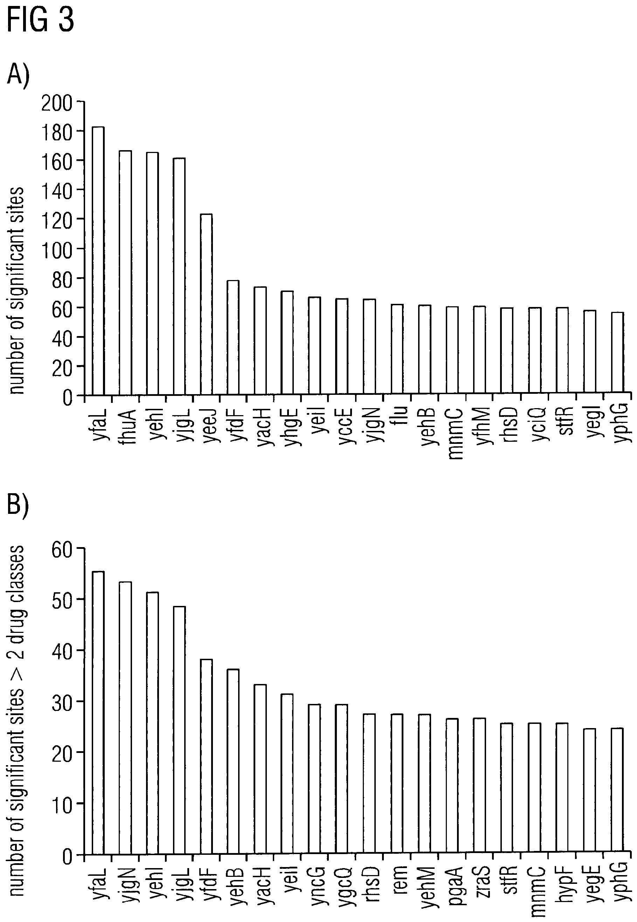

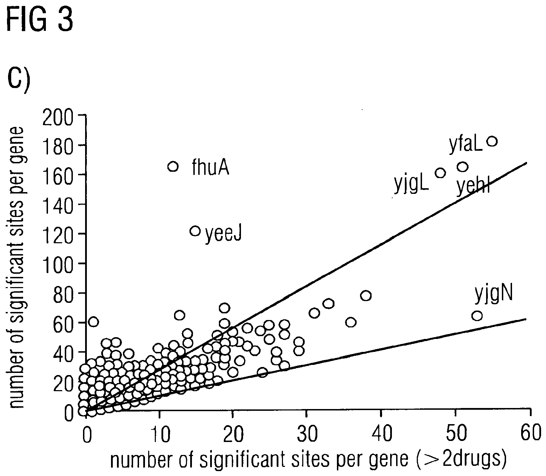

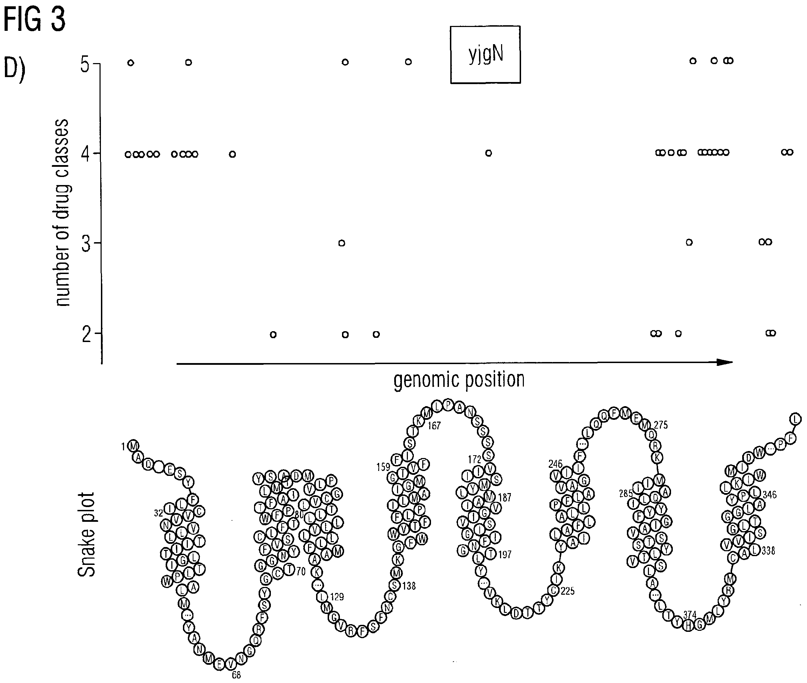

Figure 3: Panel A: bar chart of genes with highest number of significant sites. Panel B. bar chart detailing the genes with highest number of sites correlated to at least 3 drugs. Panel C. Scatter plot showing for each gene the number of significant sites correlated with at least 3 drugs as function of total number of significant sites in the gene. Colored genes represent those with highest absolute numbers (yfaL, yehL, YjgL)), with higher frequency of resistance correlated to at least 3 drugs (yjgN) and genes with lower significant sites in at least 3 drugs (fhuA, yeeJ). Panel D. Along gene plot for yigN. The significant sites along the genetic sequence are presented as dots, the y-axis shows the number of drug classes significant for the respective site. Below, a so called snake plot of the trans-membrane protein is shown, the affected amino acids are indicated.
Figure 4: Panel A: network diagram showing drugs as rectangles and genes with higher or lower coverage if resistance for the respective drug is shown as circles. mmuP, mmuM, yiel, insN-1 correspond to higher read counts in case of resistant isolates while green genes correspond to lower coverage. Panel B and C: two example along-chromosome plots. Each sample is represented by a line, black lines correspond to non-resistant and gray lines to resistant isolates.
Figure 5: Decision diagram for ampicillin.
Figure 6: Decision diagram for ampicillin sulbactam.
Figure 7: Decision diagram for amoxicillin clavulanate.
Figure 8: Decision diagram for aztreonam.
Figure 9: Decision diagram for ceftriaxone.
Figure 10: Decision diagram for ceftazidime.
Figure 11: Decision diagram for cefotaxime.
Figure 12: Decision diagram for ciprofloxacin.
Figure 13: Decision diagram for cefepime.
Figure 14: Decision diagram for cefuroxime.
Figure 15: Decision diagram for ertapenem.
Figure 16: Decision diagram for gentamycin.
Figure 17: Decision diagram for levofloxacin.
Figure 18: Decision diagram for piperazillin tazobactam.
Figure 19: Decision diagram for tobramycin.
Figure 20: Decision diagram for trimethoprim sulfmethoxazole.

### EXAMPLES

### Example 1

Here, a unique collection of genes was identified that allow the determination the resistance of a bacterial microorganism to commonly used antibiotic drugs.

A unique cohort of bacterial samples obtained from 150 clinical isolates was sequenced in order to understand the genetic resistance mechanisms by using High Throughput sequencing. In parallel, classical resistance tests were applied using 21 drugs or combinations of drugs (Table 1)

**Table 1: Antibiotic Drugs**

| Medication | Drugbank ID | Abbreviation |
|---|---|---|
| Amoxicillin | DB00766 | |
| Clavulanate | DB01060 | AUG |
| Ampicillin | DB00415 | AM |
| Ampicillin Sulbactam | DB00415 | A/S |
| Aztreonam | DB00355 | AZT |
| Cefazolin | DB01327 | CFZ |
| Cefepime | DB01413 | CPE |
| Cefotaxime | DB00493 | CFT |
| Ceftazidime | DB00438 | CAZ |
| Ceftriaxone | DB01212 | CAX |
| Cefuroxime | DB01112 | CRM |
| Cephalotin | DB00456 | CF |
| Ciprofloxacin | DB00537 | CP |
| Gentamicin | DB00798 | GM |
| Imipenem | DB01598 | IMP |
| Levofloxacin | DB01137 | LVX |
| Piperacillin | DB00319 | |
| Tazobactam | DB01606 | P/T |
| Tetracycline | DB00759 | TE |
| Tobramycin | DB00684 | TO |
| Trimethoprim | DB00440 | |
| Sulfamethoxaxole | DB01015 | T/S |
| Meropenem | DB00760 | MER |
| Ertapenem | DB00303 | ETP |

E. coli strains to be tested were seeded on agar plates and incubated under growth conditions for 24 hours. Then, colonies were picked and incubated in growth medium in the presence of a given antibiotic drug in dilution series under growth conditions for 16-20 hours. Bacterial growth was determined by observing turbidity.

Next mutations were searched that are highly correlated with the results of the phenotypic resistance test.

For sequencing, samples were prepared using a Nextera library preparation, followed by multiplexed sequencing using the Illuminat HiSeq 2500 system, paired end sequencing. Data were mapped with BWA (Li H. and Durbin R. (2010) Fast and accurate long-read alignment with Burrows-Wheeler Transform. Bioinformatics, Epub. [PMID: 20080505])and SNP were called using samtools (Li H.*, Handsaker B.*, Wysoker A., Fennell T., Ruan J., Homer N., Marth G., Abecasis G., Durbin R. and 1000 Genome Project Data Processing Subgroup (2009) The Sequence alignment/map (SAM) format and SAMtools. Bioinformatics, 25, 2078-9. [PMID: 19505943]).

The reference sequence was obtained from Escherichia coli str. K-12 substr. DH10B:
LOCUS CP000948 4686137 bp DNA circular BCT 05-JUN-2008
DEFINITION Escherichia coli str. K12 substr. DH10B, complete genome.
ACCESSION CP000948
VERSION CP000948.1 GI:169887498
DBLINK BioProject: PRJNA20079
KEYWORDS .
SOURCE Escherichia coli str. K-12 substr. DH10B
ORGANISM Escherichia coli str. K-12 substr. DH10B
   Bacteria; Proteobacteria; Gammaproteobacteria; Enterobacteriales; Enterobacteriaceae; Escherichia.

### REFERENCE 1 (bases 1 to 4686137)

AUTHORS Durfee,T., Nelson,R., Baldwin,S., Plunkett,G. III, Burland,V., Mau,B., Petrosino,J.F., Qin,X., Muzny,D.M., Ayele,M., Gibbs,R.A., Csorgo,B., Posfai,G., The inventorsin-stock,G.M. and Blattner,F.R.
TITLE The complete genome sequence of Escherichia coli DH10B: insights into the biology of a laboratory workhorse
JOURNAL J. Bacteriol. 190 (7), 2597-2606 (2008)
PUBMED 18245285

### REFERENCE 2 (bases 1 to 4686137)

AUTHORS Plunkett,G. III.
TITLE Direct Submission
JOURNAL Submitted (20-FEB-2008) Department of Genetics and Biotechnology, University of Wisconsin, 425G Henry Mall, Madison, WI 53706, USA
COMMENT DH10B and DH10B-T1R are available from Invitrogen Corporation
   (http://www.invitrogen.com).

The mutations were matched to the genes and the amino acid changes were calculated. Using different algorithms (SVM, homology modeling) mutations leading to amino acid changes with likely pathogenicity / resistance were calculated. Known variants from the swissprot database were excluded and all variants in the respective genes selected.

As noted above, for E.coli 86 ultra highly significant pairs of genetic positions and drug resistance (table 2) were identified. The 86 combinations correspond to 35 genetic positions, since the sites are usually significant for more than one single drug. Most importantly, the respective sites are located in 9 genes: hofB, allA, mukB, ymdC, potB, ycgK, ycgB, valS, yjjJ. These genes thus appear to be critical for antibiotic resistance/susceptibility. The identified mutations all lead to amino acid alterations, either to an exchange of amino acid at the respective position or the creation of a new stop-codon. Thereby, resistance related variants for the following 6 antibiotic drugs were detected: CP, LVX, TE, CFZ, CRM, GM.

**Table 2: Identified Mutations**

| Genome Pos | Therapy | p-value | gene pos | Ref | Alt | AA | Alt AA | Gene | Exchange |
|---|---|---|---|---|---|---|---|---|---|
| 90064 | CP | 2,6527E-22 | 1140 | C | T | W | C | hofB | W380C |
| 90064 | LVX | 5,2992E-20 | 1140 | C | T | W | C | hofB | W380C |
| 471036 | CP | 2,6527E-22 | 31 | G | A | E | K | allA | E11K |
| 471036 | LVX | 5,2992E-20 | 31 | G | A | E | K | allA | E11K |
| 1030161 | CP | 2,6527E-22 | 685 | C | T | R | C | mukB | R229C |
| 1030161 | LVX | 5,2992E-20 | 685 | C | T | R | C | mukB | R229C |
| 1161719 | CP | 4,7722E-13 | 697 | A | C | N | H | ymdC | N233H |
| 1161719 | LVX | 8,8353E-13 | 697 | A | C | N | H | ymdC | N233H |
| 1161764 | CP | 2,6527E-22 | 742 | C | T | R | C | ymdC | R248C |
| 1161764 | LVX | 5,2992E-20 | 742 | C | T | R | C | ymdC | R248C |
| 1238314 | CP | 3,4979E-18 | 799 | A | G | L | V | potB | L267V |
| 1238314 | LVX | 7,91E-17 | 799 | A | G | L | V | potB | L267V |
| 1238314 | TE | 2,5459E-07 | 799 | A | G | L | V | potB | L267V |
| 1239076 | CP | 2,6527E-22 | 37 | C | T | V | F | potB | V13F |
| 1239076 | LVX | 5,2992E-20 | 37 | C | T | V | F | potB | V13F |
| 1266748 | CP | 8,9009E-16 | 189 | A | G | H | Q | ycgK | H63Q |
| 1266748 | LVX | 1,2635E-14 | 189 | A | G | H | Q | ycgK | H63Q |
| 1266748 | CFZ | 4,4331E-10 | 189 | A | G | H | Q | ycgK | H63Q |
| 1266748 | TE | 6,1345E-10 | 189 | A | G | H | Q | ycgK | H63Q |
| 1266748 | CRM | 6,0841E-07 | 189 | A | G | H | Q | ycgK | H63Q |
| 1266829 | CP | 2,6527E-22 | 108 | G | A | S | R | ycgK | S36R |
| 1266829 | LVX | 5,2992E-20 | 108 | G | A | S | R | ycgK | S36R |
| 1275217 | LVX | 1,2048E-15 | 1489 | T | C | I | L | ycgB | I497L |
| 1275217 | CP | 1,5912E-15 | 1489 | T | C | I | L | ycgB | I497L |
| 1275217 | TE | 1,8029E-07 | 1489 | T | C | I | L | ycgB | I497L |
| 1275217 | CFZ | 6,7512E-07 | 1489 | T | C | I | L | ycgB | I497L |
| 1275307 | CP | 2,6527E-22 | 1399 | G | A | L | M | ycgB | L467M |
| 1275307 | LVX | 5,2992E-20 | 1399 | G | A | L | M | ycgB | L467M |
| 4582262 | CP | 3,7422E-11 | 1407 | G | A | D | E | valS | D469E |
| 4582262 | LVX | 7,4232E-10 | 1407 | G | A | D | E | valS | D469E |
| 4582262 | CFZ | 7,6762E-07 | 1407 | G | A | D | E | valS | D469E |
| 4582280 | CP | 1,1193E-12 | 1389 | A | G | D | E | valS | D463E |
| 4582280 | LVX | 1,2386E-12 | 1389 | A | G | D | E | valS | D463E |
| 4582301 | LVX | 1,6858E-16 | 1368 | T | C | K | N | valS | K456N |
| 4582301 | CP | 4,0168E-16 | 1368 | T | C | K | N | valS | K456N |
| 4582313 | LVX | 3,6594E-16 | 1356 | G | A | D | E | valS | D452E |
| 4582313 | CP | 2,3782E-15 | 1356 | G | A | D | E | valS | D452E |
| 4582337 | CP | 3,3749E-11 | 1332 | G | A | N | K | valS | N444K |
| 4582337 | LVX | 1,2081E-10 | 1332 | G | A | N | K | valS | N444K |
| 4582337 | CFZ | 3,6214E-08 | 1332 | G | A | N | K | valS | N444K |
| 4582352 | CP | 4,0453E-16 | 1317 | A | G | Y | * | valS | Y439* |
| 4582352 | LVX | 5,235E-16 | 1317 | A | G | Y | * | valS | Y439* |
| 4582352 | CFZ | 3,2243E-07 | 1317 | A | G | Y | * | valS | Y439* |
| 4582382 | CP | 3,7422E-11 | 1287 | C | T | L | F | valS | L429F |
| 4582382 | LVX | 7,4232E-10 | 1287 | C | T | L | F | valS | L429F |
| 4582430 | CP | 1,4858E-11 | 1239 | G | A | Y | * | valS | Y413* |
| 4582430 | LVX | 4,4757E-11 | 1239 | G | A | Y | * | valS | Y413* |
| 4582430 | CFZ | 2,3492E-07 | 1239 | G | A | Y | * | valS | Y413* |
| 4582838 | LVX | 1,1423E-14 | 831 | T | C | K | N | valS | K277N |
| 4582838 | CP | 1,2258E-14 | 831 | T | C | K | N | valS | K277N |
| 4582838 | CFZ | 7,2547E-08 | 831 | T | C | K | N | valS | K277N |
| 4582937 | LVX | 6,011E-18 | 732 | A | G | D | E | valS | D244E |
| 4582937 | CP | 2,912E-17 | 732 | A | G | D | E | valS | D244E |
| 4582937 | GM | 7,6578E-07 | 732 | A | G | D | E | valS | D244E |
| 4582943 | CP | 1,7589E-13 | 726 | G | A | Y | * | valS | Y242* |
| 4582943 | LVX | 1,3467E-11 | 726 | G | A | Y | * | valS | Y242* |
| 4582943 | CFZ | 1,2817E-09 | 726 | G | A | Y | * | valS | Y242* |
| 4582943 | TE | 1,3866E-07 | 726 | G | A | Y | * | valS | Y242* |
| 4582987 | CP | 1,7363E-22 | 682 | G | A | L | M | valS | L228M |
| 4582987 | LVX | 3,2E-21 | 682 | G | A | L | M | valS | L228M |
| 4583141 | CP | 3,6032E-18 | 528 | A | G | D | E | valS | D176E |
| 4583141 | LVX | 1,1539E-17 | 528 | A | G | D | E | valS | D176E |
| 4583141 | CFZ | 7,2389E-08 | 528 | A | G | D | E | valS | D176E |
| 4666362 | CP | 5,4173E-19 | 109 | G | A | D | N | yjjJ | D37N |
| 4666362 | LVX | 2,3023E-18 | 109 | G | A | D | N | yjjJ | D37N |
| 4666405 | CP | 5,4173E-19 | 152 | C | T | A | V | yjjJ | A51V |
| 4666405 | LVX | 2,3023E-18 | 152 | C | T | A | V | yjjJ | A51V |
| 4666461 | CP | 9,4295E-08 | 208 | A | G | T | A | yjjJ | T70A |
| 4666461 | LVX | 8,4496E-07 | 208 | A | G | T | A | yjjJ | T70A |
| 4666768 | CP | 9,4295E-08 | 515 | A | G | H | R | yjjJ | H172R |
| 4666768 | LVX | 8,4496E-07 | 515 | A | G | H | R | yjjJ | H172R |
| 4666804 | CP | 1,6956E-22 | 551 | A | G | H | R | yjjJ | H184R |
| 4666804 | LVX | 7,7611E-22 | 551 | A | G | H | R | yjjJ | H184R |
| 4666804 | CFZ | 4,5083E-07 | 551 | A | G | H | R | yjjJ | H184R |
| 4666885 | CP | 9,4295E-08 | 632 | A | G | Y | C | yjjJ | Y211C |
| 4666885 | LVX | 8,4496E-07 | 632 | A | G | Y | C | yjjJ | Y211C |
| 4667178 | CP | 9,4295E-08 | 925 | C | G | Q | E | yjjJ | Q309E |
| 4667178 | LVX | 8,4496E-07 | 925 | C | G | Q | E | yjjJ | Q309E |
| 4667191 | CP | 2,6527E-22 | 938 | G | A | R | H | yjjJ | R313H |
| 4667191 | LVX | 5,2992E-20 | 938 | G | A | R | H | yjjJ | R313H |
| 4667359 | CP | 9,4295E-08 | 1106 | T | C | V | A | yjjJ | V369A |
| 4667359 | LVX | 8,4496E-07 | 1106 | T | C | V | A | yjjJ | V369A |
| 4667424 | CP | 2,6527E-22 | 1171 | G | A | V | I | yjjJ | V391I |
| 4667424 | LVX | 5,2992E-20 | 1171 | G | A | V | I | yjjJ | V391I |
| 4667568 | CP | 1,2838E-17 | 1315 | G | A | A | T | yjjJ | A439T |
| 4667568 | LVX | 2,3051E-17 | 1315 | G | A | A | T | yjjJ | A439T |

In Table 2 the columns are designated as follows:
Genome Pos: genomic position of the SNP / variant in the E. coli reference genome (see below);
Therapy: the therapy to which the mutation is significantly correlated, multiple therapies are in separate rows (if a SNP is correlated to e.g. 4 therapies this leads to 4 single rows);
P-value: significance value calculated using fishers exact test;
Gene pos: position of the mutation in the gene;
Ref: reference base, A, C, T, G;
Alt: Alternative base associated with resistance;
AA: original Amino acid;
Alt A: changed amino acid;
Gene: affected gene;
Exchange: amino acid exchange in standard nomenclature;

P-value was calculated using the fisher exact test based on contingency table with 4 fields: #samples Resistant / wild type; #samples Resistant / mutant; #samples not Resistant / wild type; #samples not Resistant / mutant

In table 3, the identified genes and gene products are listed and identified by Gene ID of the gene and (NCBI) Accession number of the corresponding protein corresponding to

**Table 3: Gene name and Identifier**

| Gene name | Gene ID | Accession No. |
|---|---|---|
| hofB | 6061494 | ACB01286.1 |
| allA | 6059827 | ACB01630.1 |
| mukB | 6060547 | ACB02124.1 |
| ymdC | 6059214 | ACB02240.1 |
| potB | 6058608 | ACB02318.1 |
| ycgK | 6058586 | ACB02348.1 |
| valS | 6060190 | ACB05239.1 |
| yjjJ | 6058313 | ACB05313.1 |
| ycgB | 6058539 | ACB02358.1 |

The test is based on the distribution of the samples in the 4 fields. Even distribution indicates no significance, while clustering into two fields indicates significance.

Using this approach 35 highly significant, novel genetic positions or mutations in 9 genes (hofB, allA, mukB, ymdC, potB, ycgK, ycgB, valS, yjjJ) were identified which can be used for and allow the determination of resistance to commonly used antibiotic drugs. All the highly significant mutations described herein and listed in table 2 are non-conservative mutations leading to an amino acid exchange or a new stop-codon (designated with a "*" symbol in table 2), and thus to an altered protein. It is thus likely that the identified 9 genes play a significant role in antibiotic resistance and are putative targets for developing new drug candidates.

### Example 2

In this example, the inventors evaluate genetic susceptibility of E. coli to 21 different drugs from five drug classes (see below).
Methods: Antimicrobial susceptibility test (AST) for 1,162 clinical E. coli isolates with varying spectra of resistance to 21 FDA-approved drugs was performed and genomes of all isolates were sequenced. Genetic variants were correlated to the AST data.
Results: The inventors report 25,744 sites in the *E. coli* genome significantly correlated to drug resistance. Highest significance was reached for the drugs Ciprofloxacin and Levofloxacin with respect to amino acid (AA) exchange S83L in GyrA (p_{Ciprofloxacin}=10⁻²³⁵, accuracy, specificity and sensitivity: 98%, 99%, and 94%; p_{Levofloxacin}=10⁻²⁰⁹, 97%, 98%, 93%), a target for quinolones. The second most significant association was observed for ParC, a second target of quinolones (AA exchange S80I, p_{Ciprofloxacin}=10⁻¹⁹⁶ and p_{Levofloxacin} =10⁻¹⁹⁴). Particularly many AA exchanges significantly associated with resistance to multiple drugs were discovered in YigN. By analyzing the sequence coverage on the genome level, the inventors identified a gene dose dependency of several genes, including *mmuP* and *mmuM,* encoding a putative S-methylmethionine transporter and a homocysteine S-methyltransferase. Both loci are associated with resistance against â-lactams and quinolones.
Conclusion: The inventors here present a high-throughput screening and analysis pipeline to investigate antibiotics resistance in E. coli strains. The results demonstrate the potential of genetics-based tests to predict susceptibility against antimicrobial drugs. In addition, novel correlations of gene dose to resistance are reported.

The inventors carried out a systematic evaluation using E. coli. Specifically, the inventors collected 1,162 E. coli samples over 22 years (1991-2013) across over 60 different institutes. For these isolates, the inventors carried out standard AST for 21 FDA-approved drugs and performed Whole Genome Sequencing (WGS) for the same 1,162 isolates to build a database revealing genetic sites for predicting AST from genetic data.

### Methods

### Bacterial Strains

The inventors selected 1,162 E. coli strains from the microbiology strain collection at Siemens Healthcare Diagnostics (West Sacramento, CA) for susceptibility testing and whole genome sequencing.

### Antimicrobial Susceptibility Testing Panels

Frozen reference AST panels were prepared following Clinical Laboratory Standards Institute (CLSI) recommendations¹⁶. The following antimicrobial agents (with µg/ml concentrations shown in parentheses) were included in the panels: Amoxicillin/K Clavulanate (0.5/0.25-64/32), Ampicillin (0.25-128), Ampicillin/Sulbactam (0.5/0.25-64/32), Aztreonam (0.25-64), Cefazolin (0.5-32), Cefepime (0.25-64), Cefotaxime (0.25-128), Ceftazidime (0.25-64), Ceftriaxone (0.25-128), Cefuroxime (1-64), Cephalothin (1-64), Ciprofloxacin (0.015-8), Ertepenem (0.12-32), Gentamicin (0.12-32), Imipenem (0.25-32), Levofloxacin (0.25-16), Meropenem (0.12-32), Piperacillin/Tazobactam (0.25/4-256/4), Tetracycline (0.5-64), Tobramycin (0.12-32), and Trimethoprim/Sulfamethoxazole (0.25/4.7-32/608). Prior to use with clinical isolates, AST panels were tested with QC strains. AST panels were considered acceptable for testing with clinical isolates when the QC results met QC ranges described by CLSI16.

### Inoculum Preparation

Isolates were cultured on trypticase soy agar with 5% sheep blood (BBL, Cockeysville, Md.) and incubated in ambient air at 35±1°C for 18-24 h. Isolated colonies (4-5 large colonies or 5-10 small colonies) were transferred to a 3 ml Sterile Inoculum Water (Siemens) and emulsified to a final turbidity of a 0.5 McFarland standard. 2 ml of this suspension was added to 25 ml Inoculum Water with Pluronic-F (Siemens). Using the Inoculator (Siemens) specific for frozen AST panels, 5 µl of the cell suspension was transferred to each well of the AST panel. The inoculated AST panels were incubated in ambient air at 35±1°C for 16-20 h. Panel results were read visually, and minimal inhibitory concentrations (MIC) were determined.

### DNA extraction

Four streaks of each Gram-negative bacterial isolate cultured on trypticase soy agar containing 5% sheep blood and cell suspensions were made in sterile 1.5 ml collection tubes containing 50 µl Nuclease-Free Water (AM9930, Life Technologies). Bacterial isolate samples were stored at -20 °C until nucleic acid extraction. The Tissue Preparation System (TPS) (096D0382-02_01_B, Siemens) and the VERSANT® Tissue Preparation Reagents (TPR) kit (10632404B, Siemens) were used to extract DNA from these bacterial isolates. Prior to extraction, the bacterial isolates were thawed at room temperature and were pelleted at 2000 G for 5 seconds. The DNA extraction protocol DNAext was used for complete total nucleic acid extraction of 48 isolate samples and eluates, 50 µl each, in 4 hours. The total nucleic acid eluates were then transferred into 96-Well qPCR Detection Plates (401341, Agilent Technologies) for RNase A digestion, DNA quantitation, and plate DNA concentration standardization processes. RNase A (AM2271, Life Technologies) which was diluted in nuclease-free water following manufacturer's instructions was added to 50 µl of the total nucleic acid eluate for a final working concentration of 20 µg/ml. Digestion enzyme and eluate mixture were incubated at 37°C for 30 minutes using Siemens VERSANT® Amplification and Detection instrument. DNA from the RNase digested eluate was quantitated using the Quant-iT™ PicoGreen dsDNA Assay (P11496, Life Technologies) following the assay kit instruction, and fluorescence was determined on the Siemens VERSANT® Amplification and Detection instrument. Data analysis was performed using Microsoft® Excel 2007. 25 µl of the quantitated DNA eluates were transferred into a new 96-Well PCR plate for plate DNA concentration standardization prior to library preparation. Elution buffer from the TPR kit was used to adjust DNA concentration. The standardized DNA eluate plate was then stored at -80°C until library preparation.

### Next Generation Sequencing

Prior to library preparation, quality control of isolated bacterial DNA was conducted using a Qubit 2.0 Fluorometer (Qubit dsDNA BR Assay Kit, Life Technologies) and an Agilent 2200 TapeStation (Genomic DNA ScreenTape, Agilent Technologies). NGS libraries were prepared in 96 well format using NexteraXT DNA Sample Preparation Kit and NexteraXT Index Kit for 96 Indexes (Illumina) according to the manufacturer's protocol. The resulting sequencing libraries were quantified in a qPCR-based approach using the KAPA SYBR FAST qPCR MasterMix Kit (Peqlab) on a ViiA 7 real time PCR system (Life Technologies). 96 samples were pooled per lane for paired-end sequencing (2x 100bp) on Illumina Hiseq2000 or Hiseq2500 sequencers using TruSeq PE Cluster v3 and TruSeq SBS v3 sequncing chemistry (Illumina). Basic sequencing quality parameters were determined using the FastQC quality control tool for high throughput sequence data (Babraham Bioinformatics Institute).

### Data analysis

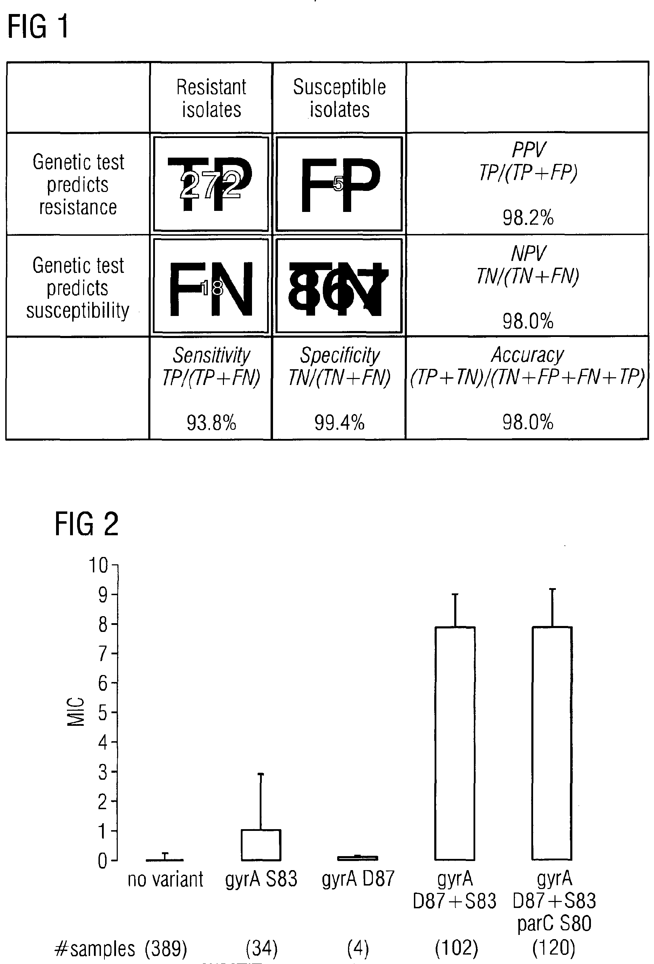

Raw paired-end sequencing data for the 1,162 E. coli samples were mapped against the E. coli DH10B reference (NC_010473) (see also above in Example 1) with BWA 0.6.1.20 The resulting SAM files were sorted, converted to BAM files, and PCR duplicates were marked using the Picard tools package 1.104 (http://picard.sourceforge.net/). The Genome Analysis Toolkit 3.1.1 (GATK)21 was used to call SNPs and indels for blocks of 200 E. coli samples (parameters: -ploidy 1 -glm BOTH -stand_call_conf 30 -stand_emit_conf 10). VCF files were combined into a single file and quality filtering for SNPs was carried out (QD < 2.0 | | FS > 60.0 | | MQ < 40.0) and indels (QD < 2.0 | | FS > 200.0). Detected variants were annotated with SnpEff22 to predict coding effects. For each annotated position, genotypes of all E. coli samples were considered. E. coli samples were split into two groups, low resistance group (having lower MIC concentration for the considered drug), and high resistance group (having higher MIC concentrations) with respect to a certain MIC concentration (breakpoint). To find the best breakpoint all thresholds were evaluated and p-values were computed with Fisher's exact test relying on a 2x2 contingency table (number of E. coli samples having the reference or variant genotype vs. number of samples belonging to the low and high resistance group). The best computed breakpoint was the threshold yielding the lowest p-value for a certain genomic position and drug. For further analyses positions with non-synonymous alterations and p-value < 10-9 were considered. Based on the contingency table, the accuracy (ACC), sensitivity (SENS), specificity (SPEC), and the positive/negative predictive values (PPV/NPV) were calculated (Figure 1).

Since a potential reason for drug resistance is gene duplication, gene dose dependency was evaluated. For each sample the genomic coverage for each position was determined using BED Tools. 23 Gene ranges were extracted from the reference assembly NC_010473.gff and the normalized median coverage per gene was calculated. To compare low- and high-resistance isolates the best area under the curve (AUC) value was computed. Groups of at least 20% of all samples having a median coverage larger than zero for that gene and containing more than 15 samples per group were considered in order to exclude artifacts and cases with AUC > 0.75 were further evaluated.

### Results

The aim of our study was to demonstrate the feasibility of genetic antimicrobial susceptibility tests (GAST), to verify our method for known resistance mechanisms, and to discover novel mechanisms. The inventors performed culture-based AST for 1,162 E. coli isolates and 21 antimicrobial drugs belonging to 5 different drug classes: â-lactams, fluoroquinolones, aminoglycosides, tetracyclines, and folate synthesis inhibitors. The complete list of drugs is shown in Table 1. For the same 1,162 E. coli isolates, whole genome sequencing using Illumina's HiSeq2500 instrument was carried out.

### Most significant sites in the E. coli genome

In order to calculate genome-wide significance scores, the inventors mapped all 1,162 *E. coli* genomes to the reference strain DH10B. For each genomic position the inventors determined the base for each sample and discovered 973,226 sites that passed the quality filtering and in which at least one sample had a non-reference base. The respective sites were correlated to the AST data for the 21 drugs using Fisher's exact test. Our analysis revealed 25,744 sites where a genetic mutation significantly correlated with at least one drug (p-value<10⁻⁹) and led to a change in the AA sequence, including point mutation and small insertions and deletions. The highest significance was reached for AA exchange S83L in GyrA and the drug Ciprofloxacin (p = 10⁻²³⁵). Remarkably, GyrA is one of the targets of Ciprofloxacin. For this position, three AA exchanges, S83L, S83W, S83A, are annotated in UniProt as conferring resistance to quinolones. For this site, only 5 false positive (0.4%) and 18 false negative samples (1.6%) were discovered while 1,139 samples were identified correctly, corresponding to accuracy, specificity, and sensitivity of 98.0%, 99.4% and 93.8%, respectively (Figure 1). Similarly, the second most significant site in GyrA, D87N / D87Y revealed just 12 false positives and 10 false negatives, the respective p-value was 10⁻²⁰⁶ and the accuracy 98.1%. Again, for this site the D87N exchange is annotated as conferring quinolone resistance in UniProt. For the third and fourth most significant sites, located in the second Ciprofloxacin target, ParC, (S80I, E84G), resistance related variants have also been described. In Figure 2, the inventors present the means and standard deviations of MICs for Ciprofloxacin for samples having no variant in GyrA (S83/D87) and ParC (S80), samples having only one mutation either in GyrA S83 or D87 and not ParC, samples having both mutations in GyrA and not ParC, and samples having all three mutations. Interestingly, the mean MIC values increase from below 1.0 for no or single mutants to above 7.8 for double or triple mutants, which shows that a combination of mutations is necessary to reach a higher level of resistance against Ciprofloxacin in this case.

Besides the mutations in type II topoisomerase drug targets (GyrA/ParC), mutations in genes *ygiF* (A110T, p=10⁻⁶⁷, acc=86%, spec=89.5%, sens=69.9%) and *ygjM* (A68V, p=10⁻⁶³, acc=89.9%, spec=94.4%, sens=67.1%) have also a high significance. Compared to the above-described AA exchanges, these two sites demonstrate a substantially decreased sensitivity and positive predictive values (PPV). While the PPV for the four AA exchanges in GyrA and ParC was between 94.8% and 98.2%, the PPV of these two exchanges decreases to 59.0% and 70.8%. This means that the likelihood to be resistant given the exchanged AA is almost as high as the likelihood to be susceptible given the exchanged AA, limiting the probability that the respective AA exchanges are causative.

To discover other AA exchanges that are potentially causative for drug resistance, the inventors filtered the list of all 25,744 sites (at least 150 resistant *E. coli* isolates carry the AA exchange, NPV>50%, PPV>75%). This filtering revealed 127 candidate sites (see also Table 4). Besides the already described exchanges in GyrA and ParC, the inventors discovered AA exchanges in YdjO associated with predicted resistance to different â-lactams (V121E, S120C, V118F, I114V, K111E, and D112N). Likewise, for lactams the inventors report AA exchanges in YcbS (E848Q, E848*), RhsC (R717Q, W492C), YcbQ (T86I), YagR (S274T) and YeaU (N293K). Finally, the inventors discovered AA exchanges related to quinolones, tetracycline, and lactams in YhaL (altogether 23 different sites). In addition, the inventors computed the most significant non-synonymous AA exchange for each drug (p-value threshold<10⁻⁹). Of 21 tested drugs, only two (Imipenem, Meropenem) were not found to be associated with an AA exchange with such a low p-value. Interestingly, the S83L mutation in GyrA is the predominant exchange in 15 drugs. For the drugs Ciprofloxacin and Levofloxacin, of which GyrA is a target, the p-values were however much lower than the p-values for this mutation in association with the remaining 13 drugs (>10⁻⁶² vs. <10⁻²⁰⁹). In addition, the inventors observed again a significant decrease in sensitivity and/or PPV in these cases: either the sensitivity or PPV is below 55% for drugs, of which GyrA is not the target, demonstrating that these measures are effective for separating mutations in true targets from others.

### Mutations in known drug targets

In 9 cases, the inventors detected mutations associated with drugs in genes that are also encoding the targets for the respective drugs. This includes the mutations associated with Ciprofloxacin and Levofloxacin in GyrA (S83L, D87N, D87Y, D678E, E574D) and ParC (S80I, E84G, E84V, E84A, A192V, Q481H, A471G, T718A, Q198H), mutations associated with Cephalotin in AmpC (K40R, I300V, T335I, A210P, Q196H, A236T, R248C), with Sulfamethoxazole in FolC (A319T, R88C, G217S), with Cefazolin in MrcB (D839E, QQQP815Q, R556C) and PbpC (L357V, V348A, A15T, A217V, Q495L, V768F, A701E, K766R, K766T, T764S, T764A, R602L, E446G, R669H, A202T) and with Ceftazidime in PbpG (A28V).

### Most affected genes and multi-drug resistant sites

Mutations are not uniformly distributed across E. coli genes: for example, yfaL, fhuA, yehI, yjgL, and yeeJ carry over 120 non-synonymous variants per gene (Figure 3A); in yfaL, as many as 182 significant exchanges were discovered. In order to discover sites that are relevant for multi-drug resistance, the inventors calculated the number of AA exchanges significant in association with at least 3 drug classes (Figure 3B) and plotted the respective site counts for each gene in Figure 3C. On average, 35% of all significant sites were associated with at least three drugs. While three genes, yfaL, yehI, and yjgL, had the highest number of AA exchanges, yjgN had a substantially increased number of sites associated with multi-drug resistance (53 of 64 sites, 83%), while yeeJ (15 of 122 sites, 12%) and fhuA (12 of 166 sites, 7%) carry fewer sites relevant for multiple drug classes than expected. In yjgN, the positions significantly associated with multiple drug classes were concentrated in the terminal regions of the gene (Figure 3D).

### Coverage analysis

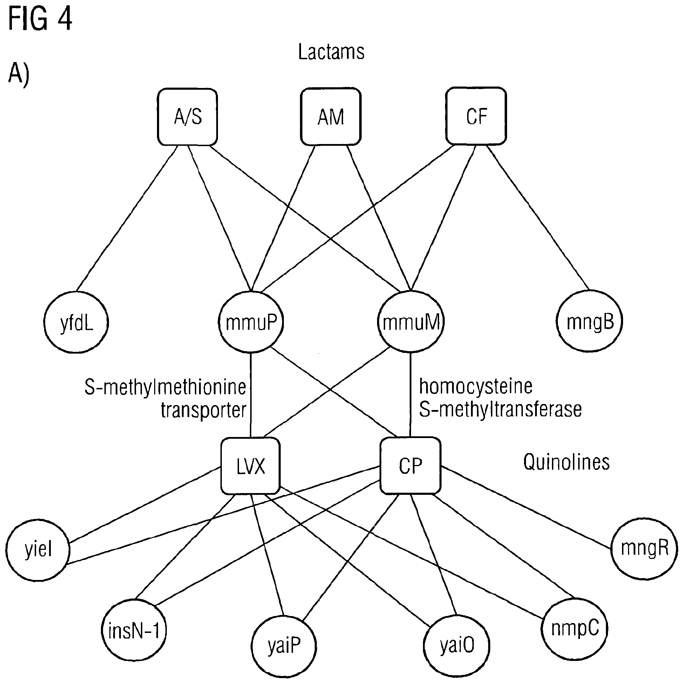

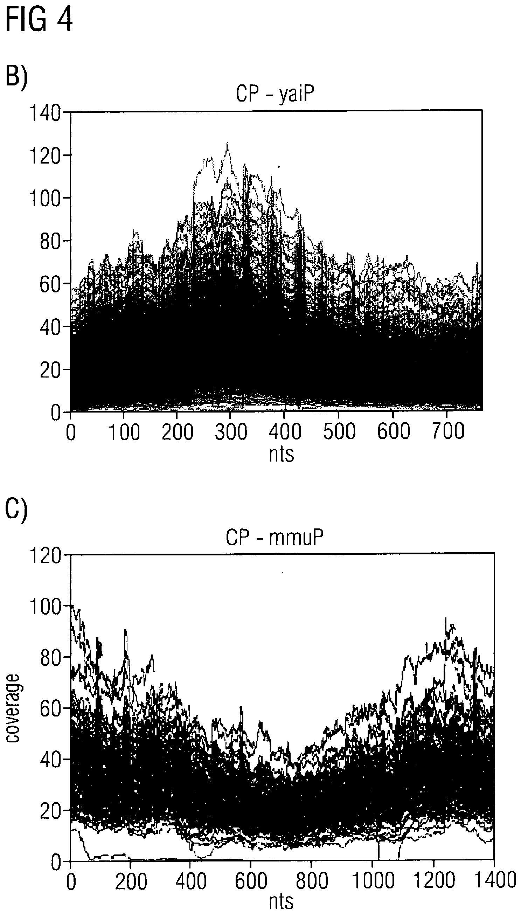

A potential reason for drug resistance is gene duplication or deletion, which can be observed in our dataset by inspecting the read coverage of different genes in the groups of resistant and susceptible isolates. To estimate the difference in coverage the inventors calculated AUC values for the normalized median coverage per gene in the two groups. Altogether the inventors discovered 23 cases of abnormal differences in gene coverage between resistant and susceptible bacteria resulting in an AUC > 0.75 (Figure 4A). The inventors report connections for three â-lactams and two quinolones. Central genes are mmuP and mmuM, encoding for a putative S-methylmethionine transporter and a homocysteine S-methyltransferase, respectively, for which the coverage is substantially higher in bacteria resistant to all 5 drugs. In strains resistant to Levofloxacin and Ciprofloxacin, the inner membrane protein YieI and InsN-1, a regulator of insertion element, were likewise higher abundant. In contrast, genes encoding glucosyltransferases YaiP, YaiO, outer membrane protein NmpC and DNA-binding transcriptional repressor MngR were less covered in strains resistant to these drugs. Figure 4B and 4C show an example coverage plot for the lower abundant covered yaiP and the higher abundant covered mmuP in strains resistant to Ciprofloxacin. Best diagnostic accuracy was reached for Ciprofloxacin and the gene mmuP, with an AUC value of 0.923, demonstrating that this quantitative information allows for accurate separation between resistant and susceptible strains.

### Discussion

The considerable and ongoing increase of infections caused by multi-drug resistant pathogens presents a major threat for patients especially in hospital settings. The development of new drugs is a long and expensive venture, and stagnated in the last years despite increasing investments in research and development. The announcement by the FDA in September 2012 to form an internal task force for supporting the development of new antimicrobial drugs emphasizes the importance of this topic. Until these drugs become available, the inventors have to learn how to apply the available ones most efficiently. Abundant prescribing of broad-spectrum antibiotics promotes the development of multi-drug resistance, so a more careful selection of drugs is needed. Thus, the inventors need methods that can quickly stratify patients and provide them with the optimal therapy. Identifying the genetic loci in the infectious agent that are predominantly responsible for an observed resistance or susceptibility is a crucial point for this.

Here, the inventors analyzed 1,162 clinical isolates of E. *coli* for their susceptibility towards 21 FDA approved drugs and combined this information with whole genome NGS data to identify potential variants that might be causative for the observed resistance patterns. In total, the inventors found 25,744 significant sites (p-value < 10⁻⁹). The method correctly identified already known drug targets in nine gene/drug combinations: *gyrA* (Ciprofloxacin, Levofloxacin), *parC* (Ciprofloxacin, Levofloxacin), *ampC* (Cephalothin), *folC* (Trimethoprim Sulfamethoxaxole), *mrcB* (Cefazolin), *pbpC* (Cefazolin), and *pbpG* (Ceftazidime). To identify other potential sites that might be secondary drug targets, the inventors applied filtering criteria using the measures NPV/PPV which allowed the inventors to reduce the number of potentially relevant sites from 25,744 to 127 sites.

Considering the best drug-target combinations according to the computed p-values, the inventors found the AA exchange S83L in GyrA to be the predominant mutation for 15 drugs. Since only Ciprofloxacin or Levofloxacin are approved drugs for GyrA, the other associations to this protein might be a side-effect of multi-drug resistance. The inventors showed that employing additional measures such as sensitivity, PPV, and NPV facilitates the separation of causative drug targets from other variants as exemplified in this case.

Instead of using only single variants, a combination of several variant positions can improve the prediction accuracy and further reduce false positive findings that are influenced by other factors.

Since gene duplication and/or deletion might also play a role in resistance development mechanisms, the inventors analyzed the gene coverage combined with the resistance data and discovered 23 cases of abnormal differences in gene coverage between resistant and susceptible bacteria. Interestingly, the inventors do not only find an increase of genetic material in resistant bacteria, e.g. for genes *mmuP, mmuM,* and *yieI*, but also a decrease in certain genes such as *mngB* and *mngR*. While for membrane or transporter proteins both an increase or a decrease of gene dosage can influence drug susceptibility by not allowing a drug to permeate the membranes or to more efficiently transport it out of the cell, a decrease of the quantity of metabolic enzymes or transcription factors is not as easily interpretable in this context, and might be more or less directly related to the fitness of the isolates.

Another source of information that might improve the accuracy of our analysis are the strain-specific plasmids. Mapping the sequencing data against those plasmids will extend our knowledge about additional resistance mechanisms. In a first approach, the inventors mapped a subset of sequencing data to about 300 *E. coli* plasmids. Among the genes having the most significant variant sites were e.g. *repA1, trbI, psiB,* and *traG* that are directly involved in replication, plasmid transfer, and maintenance and might play an indirect role in resistance development by giving its host the ability to facilitate spreading of resistance genes.

Compared to approaches using MALDI-TOF MS, the present approach has the advantage that it covers almost the complete genome and thus enables us to identify the potential genomic sites that might be related to resistance. While MALDI-TOF MS can also be used to identify point mutations in bacterial proteins³³, this technology only detects a subset of proteins and of these not all are equally well covered. In addition, the identification and differentiation of certain related strains is not always feasible.

The present method allows to compute a best breakpoint for the separation of isolates into resistant and susceptible groups. The inventors designed a flexible software tool that allows to consider besides the best breakpoints also values defined by different guidelines (e.g. European and US guidelines), preparing for an application of the GAST in different countries.

Another critical point of this study is that it analysis only included cultured bacteria strains. Several studies used culture-independent samples from urine, fecal samples, or vaginal swab and applied NGS to identify or characterize the pathogens directly. The advance of the NGS technology, including the development of new long read sequencers as PacBio and Oxford Nanopore, will further improve and speed up our procedure in the future to develop a culture-independent diagnostic test based on NGS data.

The inventors demonstrate that the present approach is capable of identifying mutations in genes that are already known as drug targets, as well as detecting potential new target sites.

### References

1. Didelot X, Bowden R, Wilson DJ, Peto TE, Crook DW. Transforming clinical microbiology with bacterial genome sequencing. Nat Rev Genet 2012;13:601-12.
2. Beerenwinkel N, Schmidt B, Walter H, et al. Diversity and complexity of HIV-1 drug resistance: a bioinformatics approach to predicting phenotype from genotype. Proc Natl Acad Sci U S A 2002;99:8271-6.
3. Reuter S, Ellington MJ, Cartwright EJ, et al. Rapid bacterial whole-genome sequencing to enhance diagnostic and public health microbiology. JAMA internal medicine 2013;173:1397-404.
4. Wozniak M, Tiuryn J, Wong L. An approach to identifying drug resistance associated mutations in bacterial strains. BMC Genomics 2012;13 Suppl 7:S23.
5. Stoesser N, Batty EM, Eyre DW, et al. Predicting antimicrobial susceptibilities for Escherichia coli and Klebsiella pneumoniae isolates using whole genomic sequence data. The Journal of antimicrobial chemotherapy 2013;68:2234-44.
6. Liu YF, Yan JJ, Lei HY, et al. Loss of outer membrane protein C in Escherichia coli contributes to both antibiotic resistance and escaping antibody-dependent bactericidal activity. Infection and immunity 2012;80:1815-22.
7. Johnson TJ, Siek KE, Johnson SJ, Nolan LK. DNA sequence and comparative genomics of pAPEC-O2-R, an avian pathogenic Escherichia coli transmissible R plasmid. Antimicrobial agents and chemotherapy 2005;49:4681-8.

## Claims

1. A method of determining an antibiotic resistance profile for a bacterial microorganism belonging to the species *E. coli* comprising:
determining the presence of a mutation in a gene of the bacterial microorganism contained or suspected of being contained in a sample provided, wherein the gene is selected from the group consisting of potB, ycgK, ycgB, valS, yjjJ, yigN, yfaL, yjgN, yehI, yjgL, yfdF, yehB, yacH, yeil, yncG, ygcQ, rhsD, rem, yehM, pgaA, zraS, stfR, mnmC, hypF, yegE, yphG, fhuA, and yeeJ,
wherein the presence of a mutation in the gene is indicative of a resistance to antibiotic drugs belonging to different groups of antibiotic drugs.

2. The method of claim 1, wherein the presence of a mutation in a gene selected from the group consisting of potB, ycgK, ycgB, valS and yjjJ is determined.

3. The method of claim 1 or 2, wherein the method comprises determining the presence of a mutation in more than one gene selected from said group.

4. The method of any one of claims 1 to 3, wherein the mutation in the gene potB is L267V.

5. The method of claim 4, wherein the mutation is indicative of a resistance to ciprofloxacin (CP), levofloxacin (LVX), and tetracycline (TE).

6. The method of any one of claims 1 to 3, wherein the mutation in the gene ycgK is H63Q.

7. The method of claim 6, wherein the mutation is indicative of a resistance to ciprofloxacin (CP), levofloxacin (LVX), cefazolin (CFZ), tetracycline (TE), and cefuroxime (CRM).

8. The method of any one of claims 1 to 3, wherein the mutation in the gene ycgB is I497L.

9. The method of claim 8, wherein the mutation is indicative of a resistance to ciprofloxacin (CP), levofloxacin (LVX), cefazolin (CFZ), and tetracycline (TE).

10. The method of any one of claims 1 to 3, wherein the mutation in the gene valS is selected from the group consisting of D469E, N444K, Y439*, Y413*, K277N, D244E, Y242* and D176E, and wherein the asterisk designates a non-conservative mutation leading to a new stop codon.

11. The method of claim 10, wherein a mutation selected from the group consisting of D469E, N444K, Y439*, Y413*, K277N, and D176E is indicative of a resistance to ciprofloxacin (CP), levofloxacin (LVX), and cefazolin (CFZ).

12. The method of claim 10, wherein the mutation D244E is indicative of a resistance to ciprofloxacin (CP), levofloxacin (LVX), and gentamicin (GM).

13. The method of claim 10, wherein the mutation Y242* is indicative of a resistance to ciprofloxacin (CP), levofloxacin (LVX), cefazolin (CFZ), and tetracycline (TE).

14. The method of any one of claims 1 to 3, wherein the mutation in the gene yjjJ is H184R.

15. The method of claim 14, wherein the mutation is indicative of a resistance to ciprofloxacin (CP), levofloxacin (LVX), and cefazolin (CFZ).

16. The method of any one of claims 1 to 15, wherein the presence of a mutation in a gene selected from said group is tested in relation to the reference strain *E. coli* K12 substrain DH10B.

## Patentansprüche

1. Verfahren zur Bestimmung eines Antibiotikaresistenzprofils für einen bakteriellen Mikroorganismus, der zu der Spezies *E. coli* gehört, das umfasst:
Bestimmung des Vorliegens einer Mutation in einem Gen des bakteriellen Mikroorganismus, der in einer bereitgestellten Probe enthalten ist oder mutmaßlich enthalten ist, wobei das Gen aus der Gruppe, bestehend aus potB, ycgK, ycgB, valS, yjjJ, yigN, yfaL, yjgN, yehI, yjgL, yfdF, yehB, yacH, yeiI, yncG, ygcQ, rhsD, rem, yehM, pgaA, zraS, stfR, mnmC, hypF, yegE, yphG, fhuA und yeeJ, ausgewählt ist,
wobei das Vorliegen einer Mutation in dem Gen auf eine Resistenz gegen antibiotische Medikamente, die zu unterschiedlichen Gruppen antibiotischer Medikamente gehören, hinweist.

2. Verfahren nach Anspruch 1, wobei das Vorliegen einer Mutation in einem Gen, das aus der Gruppe, bestehend aus potB, ycgK, ycgB, valS und yjjJ, ausgewählt ist, bestimmt wird.

3. Verfahren nach Anspruch 1 oder 2, wobei das Verfahren eine Bestimmung des Vorliegens einer Mutation in mehr als einem Gen, das aus der Gruppe ausgewählt ist, umfasst.

4. Verfahren nach einem der Ansprüche 1 bis 3, wobei die Mutation in dem Gen potB L267V ist.

5. Verfahren nach Anspruch 4, wobei die Mutation auf eine Resistenz gegen Ciprofloxacin (CP), Levofloxacin (LVX) und Tetracyclin (TE) hinweist.

6. Verfahren nach einem der Ansprüche 1 bis 3, wobei die Mutation in dem Gen ycgK H63Q ist.

7. Verfahren nach Anspruch 6, wobei die Mutation auf eine Resistenz gegen Ciprofloxacin (CP), Levofloxacin (LVX), Cefazolin (CFZ), Tetracyclin (TE) und Cefuroxim (CRM) hinweist.

8. Verfahren nach einem der Ansprüche 1 bis 3, wobei die Mutation in dem Gen ycgB I497L ist.

9. Verfahren nach Anspruch 8, wobei die Mutation auf eine Resistenz gegen Ciprofloxacin (CP), Levofloxacin (LVX), Cefazolin (CFZ) und Tetracyclin (TE) hinweist.

10. Verfahren nach einem der Ansprüche 1 bis 3, wobei die Mutation in dem Gen valS aus der Gruppe, bestehend aus D469E, N444K, Y439*, Y413*, K277N, D244E, Y242* und D176E, ausgewählt ist und wobei der Stern eine nicht-konservative Mutation, die zu einem neuen Stopcodon führt, kennzeichnet.

11. Verfahren nach Anspruch 10, wobei eine Mutation, die aus der Gruppe, bestehend aus D469E, N444K, Y439*, Y413*, K277N und D176E, ausgewählt ist, auf eine Resistenz gegen Ciprofloxacin (CP), Levofloxacin (LVX) und Cefazolin (CFZ) hinweist.

12. Verfahren nach Anspruch 10, wobei die Mutation D244E auf eine Resistenz gegen Ciprofloxacin (CP), Levofloxacin (LVX) und Gentamicin (GM) hinweist.

13. Verfahren nach Anspruch 10, wobei die Mutation Y242* auf eine Resistenz gegen Ciprofloxacin (CP), Levofloxacin (LVX), Cefazolin (CFZ) und Tetracyclin (TE) hinweist.

14. Verfahren nach einem der Ansprüche 1 bis 3, wobei die Mutation in dem Gen yjjJ H184R ist.

15. Verfahren nach Anspruch 14, wobei die Mutation auf eine Resistenz gegen Ciprofloxacin (CP), Levofloxacin (LVX) und Cefazolin (CFZ) hinweist.

16. Verfahren nach einem der Ansprüche 1 bis 15, wobei das Vorliegen einer Mutation in einem Gen, das aus der Gruppe ausgewählt ist, in Bezug auf den Referenzstamm *E. coli* K12 Unterstamm DH10B getestet wird.

## Revendications

1. Procédé de détermination d'un profil de résistance aux antibiotiques pour un microorganisme bactérien appartenant à l'espèce *E. coli* comprenant :
la détermination de la présence d'une mutation dans un gène du microorganisme bactérien contenu ou suspecté d'être contenu dans un échantillon mis à disposition, où le gène est sélectionné dans le groupe consistant en potB, ycgK, ycgB, valS, yjjJ, yigN, yfaL, yjgN, yehI, yjgL, yfdF, yehB, yacH, yeiI, yncG, ygcQ, rhsD, rem, yehM, pgaA, zraS, stfR, mnmC, hypF, yegE, yphG, fhuA, et yeeJ,
dans lequel la présence d'une mutation dans le gène est indicative d'une résistance à des médicaments antibiotiques appartenant à différents groupes de médicaments antibiotiques.

2. Procédé selon la revendication 1, dans lequel la présence d'une mutation dans un gène sélectionné dans le groupe consistant en potB, ycgK, ycgB, valS et yjjJ est déterminée.

3. Procédé selon la revendication 1 ou 2, où le procédé comprend la détermination de la présence d'une mutation dans plusieurs gènes sélectionnés dans ledit groupe.

4. Procédé selon l'une quelconque des revendications 1 à 3, dans lequel la mutation dans le gène potB est L267V.

5. Procédé selon la revendication 4, dans lequel la mutation est indicative d'une résistance à la ciprofloxacine (CP), la lévofloxacine (LVX), et la tétracycline (TE).

6. Procédé selon l'une quelconque des revendications 1 à 3, dans lequel la mutation dans le gène ycgK est H63Q.

7. Procédé selon la revendication 6, dans lequel la mutation est indicative d'une résistance à la ciprofloxacine (CP), la lévofloxacine (LVX), la céfazoline (CFZ), la tétracycline (TE), et le céfuroxime (CRM).

8. Procédé selon l'une quelconque des revendications 1 à 3, dans lequel la mutation dans le gène ycgB est 1497L.

9. Procédé selon la revendication 8, dans lequel la mutation est indicative d'une résistance à la ciprofloxacine (CP), la lévofloxacine (LVX), la céfazoline (CFZ), et la tétracycline (TE).

10. Procédé selon l'une quelconque des revendications 1 à 3, dans lequel la mutation dans le gène valS est sélectionnée dans le groupe consistant en D469E, N444K, Y439*, Y413*, K277N, D244E, Y242* et D176E, l'astérisque désignant une mutation non conservatrice entraînant un nouveau codon stop.

11. Procédé selon la revendication 10, dans lequel une mutation sélectionnée dans le groupe consistant en D469E, N444K, Y439*, Y413*, K277N, et D176E est indicative d'une résistance à la ciprofloxacine (CP), la lévofloxacine (LVX), et la céfazoline (CFZ).

12. Procédé selon la revendication 10, dans lequel la mutation D244E est indicative d'une résistance à la ciprofloxacine (CP), la lévofloxacine (LVX), et la gentamicine (GM).

13. Procédé selon la revendication 10, dans lequel la mutation Y242* est indicative d'une résistance à la ciprofloxacine (CP), la lévofloxacine (LVX), la céfazoline (CFZ), et la tétracycline (TE).

14. Procédé selon l'une quelconque des revendications 1 à 3, dans lequel la mutation dans le gène yjjJ est H184R.

15. Procédé selon la revendication 14, dans lequel la mutation est indicative d'une résistance à la ciprofloxacine (CP), la lévofloxacine (LVX), et la céfazoline (CFZ).

16. Procédé selon l'une quelconque des revendications 1 à 15, dans lequel la présence d'une mutation dans un gène sélectionné dans ledit groupe est testée par rapport à la souche de référence *E. coli* K12 sous-souche DH10B.
